# EUROPEAN PATENT APPLICATION

(11) **EP 1 121 939 A2**
(43) Date of publication of application: **08.08.2001**
(21) Application number: 01300999.8
(22) Date of filing: 05.02.2001
(51) Int. Cl.: A61K 45/06

(54) **Treatment of osteoporosis with EP2/EP4 receptor selective agonists**

(30) Priority: 07.02.2000 US 180635 P
(71) Applicant: Pfizer Products Inc., Groton, Connecticut 06340 (US)
(72) Inventor: Cameron, Kimberly O'Keefe, Groton, Connecticut 06340 (US); Thompson, David Duane, Groton, Connecticut 06340 (US); Lefker, Bruce Allen, Groton, Connecticut 06340 (US); Ke, Hua Zhu, Groton, Connecticut 06340 (US)
(74) Representative: Hayles, James Richard

(57) **Abstract**

This invention is directed to methods and pharmaceutical compositions comprising prostaglandin agonists which are useful to prevent bone loss, restore or augment bone mass and to enhance bone healing including the treatment of conditions which present with low bone mass, such as osteoporosis, and/or bone defects in vertebrates, and particularly mammals, including humans, This invention specifically relates to methods and pharmaceutical compositions comprising combinations of EP₂ receptor selective agonist and EP₄ receptor selective agonists and to methods and pharmaceutical compositions comprising agents which are agonists for both the EP₂ receptor and the EP₄ receptor.

## Description

### BACKGROUND OF INVENTION

This invention relates to methods and pharmaceutical compositions comprising prostaglandin agonists which are useful to prevent bone loss, restore or augment bone mass and enhance bone healing including the treatment of conditions which present with low bone mass and/or bone defects in vertebrates, and particularly mammals, including humans. This invention specifically relates to methods and pharmaceutical compositions comprising EP₂ receptor selective agonists and EP₄ receptor selective agonists and to methods and pharmaceutical compositions comprising agents which are both EP₂ receptor selective and EP₄ receptor selective.

Osteoporosis is a systemic skeletal disease, characterized by low bone mass and deterioration of bone tissue, with a consequent increase in bone fragility and susceptibility to fracture. In the U.S., the condition affects more than 25 million people and causes more than 1.3 million fractures each year, including 500,000 spine, 250,000 hip and 240,000 wrist fractures annually. Hip fractures are the most serious consequence of osteoporosis, with 5-20% of patients dying within one year, and over 50% of survivors being incapacitated.

The elderly are at greatest risk of osteoporosis, and the problem is therefore predicted to increase significantly with the aging of the population. Worldwide fracture incidence is forecasted to increase three-fold over the next 60 years, and one study has estimated that there will be 4.5 million hip fractures worldwide in 2050.

Women are at greater risk of osteoporosis than men. Women experience a sharp acceleration of bone loss during the five years following menopause. Other factors that increase the risk include smoking, alcohol abuse, a sedentary lifestyle and low calcium intake.

There are currently two main types of pharmaceutical therapy for the treatment of osteoporosis. The first is the use of anti-resorptive compounds to reduce the resorption of bone tissue.

Estrogen is an example of an anti-resorptive agent. It is known that estrogen reduces fractures. In addition, Black, et al. in EP 0605193A1 report that estrogen, particularly when taken orally, lowers plasma levels of LDL and raises those of the beneficial high density lipoproteins (HDL's). However, estrogen failed to restore bone back to young adult levels in the established osteoporotic skeleton. Furthermore, long-term estrogen therapy has been implicated in a variety of disorders including an increase in the risk of uterine cancer, endometrial cancer and possibly breast cancer, causing many women to avoid this treatment. The significant undesirable effects associated with estrogen therapy support the need to develop alternative therapies for osteoporosis that have the desirable effect on serum LDL but do not cause undesirable effects.

A second type of pharmaceutical therapy for the treatment of osteoporosis is the use of anabolic agents to promote bone formation and increase bone mass. This class of agents is expected to restore bone to the established osteoporotic skeleton.

U.S. pat. no. 4,112,236 discloses certain interphenylene 8-aza-9-dioxothia-11,12-secoprostaglandins for the treatment of patients with renal impairment.

Certain prostagladin agonists are disclosed in GB 1478281, GB1479156 and U.S. pat. nos. 4,175,203; 4,055,596; 4,175,203; 3,987,091 and 3,991,106 as being useful as, for example, renal vasodilators.

U.S. pat. no. 4,033,996 discloses certain 8-aza-9-oxo(and dioxo)-thia-11,12-secoprostaglandins which are useful as renal vasodilators, for the prevention of thrombus formation, to induce growth hormone release, and as regulators of the immune response.

French patent no. 897,566 discloses certain amino acid derivatives for the treatment of neurological, mental or cardiovascular disease.

U.S. pat. no. 4,761,430 discloses certain arylbenzenesulfonamide compounds as lipid-lowering agents.

U.S. pat. no. 4,443,477 discloses certain sulphonamidophenylcarboxylic acids as lipid lowering agents.

U.S. pat. no. 3,528,961 discloses certain ε-caprolactam derivatives as dyes.

U.S. pat. no. 3,780,095 discloses certain acylated anilinocarboxylic acids as choleretics.

U.S. pat. no. 4,243,678 discloses certain acylhydrocarbylaminoalkanoic acids as having utility in the treatment of gastric ulcers, as sebaceous gland excretion inhibitors and for combatting skin inflammation.

U.S. pat. no. 4,386,031 discloses certain N-benzoyl-ω-anilinoalkanecarboxylic acids as antiallergic agents, thrombotic aggregation inhibitors, antiinflammatory agents and lipid-lowering agents.

In addition to osteoporosis, approximately, 20-25 million women and an increasing number of men have detectable vertebral fractures as a consequence of reduced bone mass, with an additional 250,000 hip fractures reported yearly in America alone. The latter case is associated with a 12% mortality rate within the first two years and with a 30% rate of patients requiring nursing home care after the fracture. While this is already significant, the economic and medical consequences of convalescence due to slow or imperfect healing of these bone fractures is expected to increase, due to the aging of the general population.

Estrogens have been shown (Bolander et al., 38th Annual Meeting Orthopedic Research Society, 1992) to improve the quality of the healing of appendicular fractures. Therefore, estrogen replacement therapy might appear to be a method for the treatment of fracture repair. However, patient compliance with estrogen therapy is relatively poor due to its side effects, including the resumption of menses, mastodynia, an increased risk of uterine cancer, an increased perceived risk of breast cancer, and the concomitant use of progestins. In addition, men are likely to object to the use of estrogen treatment. The need exists for a therapy which would be beneficial to patients who have suffered debilitating bone fractures and which would increase patient compliance.

Although there are a variety of osteoporosis therapies, there is a continuing need and a continuing search in this field of art for alternative osteoporosis therapies. In addition, there is a need for bone fracture healing therapies. Also, there is a need for therapy which can promote bone re-growth into skeletal areas where defects exist such as defects caused or produced by, for example, tumors in bone. Further, there is a need for therapy which can promote bone re-growth into skeletal areas where bone grafts are indicated.

### SUMMARY OF THE INVENTION

This invention is directed to pharmaceutical compositions comprising an EP₂ receptor selective agonist, a prodrug thereof or a pharmaceutically acceptable salt of said EP₂ receptor selective agonist or of said prodrug, and an EP₄ receptor selective agonist, a prodrug thereof or a pharmaceutically acceptable salt of said EP₄ receptor selective agonist or of said prodrug. It is preferred that such pharmaceutical compositions additionally comprise a pharmaceutically acceptable vehicle, carrier or diluent.

This invention is also directed to methods of treating a condition which presents with low bone mass in a mammal comprising administering to said mammal a pharmaceutical composition as described above. In one of the preferred embodiments of the methods of this invention, the composition is administered systemically. In another preferred embodiment of the methods of this invention, the composition is administered locally. Conditions which present with low bone mass which are treated by the compositions, methods and kits of this invention include, but are not limited to, osteoporosis, osteoporotic fractures, bone defects, childhood idiopathic bone loss, alveolar bone loss, mandibular bone loss, bone fracture, osteotomy, bone loss associated with periodontitis and prosthetic ingrowth.

This invention is also directed to methods of treating a condition which presents with low bone mass in a mammal comprising administering to said mammal a an EP₄ receptor selective agonist, a prodrug thereof or a pharmaceutically acceptable salt of said EP4 receptor selective agonist or of said prodrug, and an EP₂ receptor selective agonist, a prodrug thereof or a pharmaceutically acceptable salt of said EP₂ receptor selective agonist, or of said prodrug.

This invention is especially directed to methods wherein the EP₄ receptor selective agonist, prodrug thereof or pharmaceutically acceptable salt of said EP4 receptor selective agonist or of said prodrug, and the EP₂ receptor selective agonist, prodrug thereof or pharmaceutically acceptable salt of said EP₂ receptor selective agonist or of said prodrug, are administered separately and in any order.

This invention is also especially directed to methods wherein the the EP₄ receptor selective agonist, prodrug thereof or pharmaceutically acceptable salt of said EP4 receptor selective agonist or of said prodrug, and the EP₂ receptor selective agonist, prodrug thereof or pharmaceutically acceptable salt of said EP₂ receptor selective agonist or of said prodrug, are administered together.

This invention is also directed to methods of treating a condition which presents with low bone mass in a mammal comprising administering to said mammal an effective amount of an EP₂/EP₄ receptor selective agonist, a prodrug thereof or a pharmaceutically acceptable salt of thereof or of said prodrug.

This invention is also directed to kits comprising:
a) a first unit dosage form comprising an EP₂ receptor selective agonist, a prodrug thereof or a pharmaceutically acceptable salt of said EP₂ receptor selective agonist or of said prodrug, and a pharmaceutically acceptable carrier, vehicle or diluent;
b) a second unit dosage form comprising an EP₄ receptor selective agonist, a prodrug thereof or a pharmaceutically acceptable salt of said EP₄ receptor selective agonist or of said prodrug, and a pharmaceutically acceptable carrier, vehicle or diluent; and
c) a container.

Preferably post-menopausal women and men over the age of 60 are treated. Also preferred is treatment of individuals, regardless of age, who have significantly reduced bone mass, i.e., greater than or equal to 1.5 standard deviations below young normal levels.

Methods for treating "secondary osteoporosis" are also included within the methods of this invention. "Secondary osteoporosis" includes glucocorticoid-induced osteoporosis, hyperthyroidism-induced osteoporosis, immobilization-induced osteoporosis, heparin-induced osteoporosis and immunosuppressive-induced osteoporosis in a vertebrate, e.g., a mammal (including a human being). Said treatment is achieved by administering to said vertebrate, e.g., a mammal, suffering from "secondary osteoporosis," a "secondary osteoporosis" effective treating amount of a pharmaceutical composition comprising an EP₂/EP₄ receptor selective agonist, a prodrug thereof or a pharmaceutically acceptable salt of said EP₂/EP₄ receptor selective agonist or said prodrug or by administering to said vertebrate, e.g., mammal, a "secondary osteoporosis" treating effective amount of an EP₄ receptor selective agonist, a prodrug thereof or a pharmaceutically acceptable salt of said EP₄ receptor selective agonist or of said prodrug and an EP₂ receptor selective agonist, a prodrug thereof or a pharmaceutically acceptable salt of said EP₂ receptor selective agonist or of said prodrug.

Yet another aspect of this invention is directed to methods for strengthening a bone graft, inducing vertebral synostosis, enhancing long bone extension, enhancing bone healing following facial reconstruction, maxillary reconstruction or mandibular reconstruction in a vertebrate, e.g., a mammal (including a human being), comprising administering to said vertebrate, e.g., a mammal which has undergone facial reconstruction, maxillary reconstruction or mandibular reconstruction, a bone enhancing amount of a pharmaceutical composition comprising an EP₂/EP₄ receptor selective agonist, a prodrug thereof or a pharmaceutically acceptable salt of said EP₂/EP₄ receptor selective agonist or said prodrug or by administering to said vertebrate, e.g., mammal, an EP₄ receptor selective agonist, a prodrug thereof or a pharmaceutically acceptable salt of said EP₄ receptor selective agonist or of said prodrug, and an EP₂ receptor selective agonist, a prodrug thereof or a pharmaceutically acceptable salt of said EP₂ receptor selective agonist or of said prodrug. Whether administered separately or together, the active agents of this invention may be applied locally to the site of bone reconstruction or may be administered systemically.

A preferred dosage is about 0.001 to 100 mg/kg/day of a Formula I compound, a prodrug thereof or a pharmaceutically acceptable salt of said compound or said prodrug. An especially preferred dosage is about 0.01 to 10 mg/kg/day of a Formula I compound, a prodrug thereof or a pharmaceutically acceptable salt of said compound or said prodrug.

The phrase "condition(s) which presents with low bone mass" refers to a condition where the level of bone mass is below the age specific normal as defined in standards by the World Health Organization "Assessment of Fracture Risk and its Application to Screening for Postmenopausal Osteoporosis (1994). Report of a World Health Organization Study Group, World Health Organization Technical Series 843". Included in "condition(s) which presents with low bone mass" are primary and secondary osteoporosis. Secondary osteoporosis includes glucocorticoid-induced osteoporosis, hyperthyroidism-induced osteoporosis, immobilization-induced osteoporosis, heparin-induced osteoporosis and immunosuppressive-induced osteoporosis. Also included is periodontal disease, alveolar bone loss, post-osteotomy and childhood idiopathic bone loss. The phrase "condition(s) which presents with low bone mass" also includes long term complications of osteoporosis such as curvature of the spine, loss of height and prosthetic surgery.

The phrase "condition(s) which presents with low bone mass" also refers to a vertebrate, e.g., a mammal, known to have a significantly higher than average chance of developing such diseases as are described above including osteoporosis (e.g., post-menopausal women, and men over the age of 60).

Other bone mass augmenting or enhancing uses include bone restoration, increasing the bone fracture healing rate, replacing bone graft surgery entirely, enhancing the rate of successful bone grafts, bone healing following facial reconstruction or maxillary reconstruction or mandibular reconstruction, prosthetic ingrowth, vertebral synostosis and long bone extension.

The compounds and compositions of this invention may also be used in conjunction with orthopedic devices such as spinal fusion cages, spinal fusion hardware, internal and external bone fixation devices, screws and pins.

Those skilled in the art will recognize that the term bone mass actually refers to bone mass per unit area which is sometimes (although not strictly correctly) referred to as bone mineral density.

The term "treating", "treat" or "treatment" as used herein includes preventative (e.g., prophylactic), palliative and curative treatment.

By "pharmaceutically acceptable" it is meant the carrier, vehicle, diluent, excipients, and/or salt must be compatible with the other ingredients of the formulation, and not deleterious to the recipient thereof.

The expression "prodrug" refers to a compound that is a drug precursor which, following administration, releasess the drug *in vivo* via some chemical or physiological process (e.g., a prodrug on being brought to the physiological pH or through enzyme action is converted to the desired drug form). Exemplary prodrugs upon cleavage release the corresponding drug compounds.

The expression "pharmaceutically acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate. The expression also refers to nontoxic cationic salts such as (but not limited to) sodium, potassium, calcium, magnesium, ammonium or protonated benzathine (N,N'-dibenzylethylenediamine), choline, ethanolamine, diethanolamine, ethylenediamine, meglamine (N-methyl-glucamine), benethamine (N-benzylphenethylamine), piperazine and tromethamine (2-amino-2-hydroxymethyl-1,3-propanediol).

The compositions, methods and kits of this invention result in bone formation resulting in decreased fracture rates. This invention makes a significant contribution to the art by providing compositions, methods and kits that increase bone formation resulting in prevention, retardation, and/or regression of osteoporosis and related bone disorders.

Other features and advantages will be apparent from this description and the appendant claims which describe the invention.

### DETAILED DESCRIPTION OF THE INVENTION

Any EP₂ receptor selective agonist may be used as the EP₂ receptor selective agonist of this invention. Preferred EP₂ receptor selective agonists include:
(i) compounds of Formula I 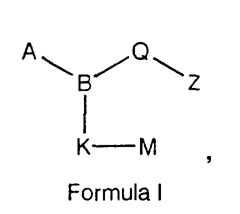 prodrugs thereof, and the pharmaceutically acceptable salts of the compounds and the prodrugs, wherein:
   B is N;
   A is (C₁-C₆)alkylsulfonyl, (C₃-C₇)cycloalkylsulfonyl, (C₃-C₇)cycloalkyl(C₁-C₆)alkylsulfonyl, said A moieties optionally mono-, di- or tri- substituted on carbon independently with hydroxy, (C₁-C₄)alkyl or halo;
   Q is
      -(C₂-C₆)alkylene-W-(C₁-C₃)alkylene-,
      -(C₃-C₈)alkylene-, said -(C₃-C₈)alkylene- optionally substituted with up to four substituents independently selected from fluoro or (C₁-C₄)alkyl,
      -X-(C₁-C₅)alkylene-,
      -(C₁-C₅)alkylene-X-,
      -(C₁-C₃)alkylene-X-(C₁-C₃)alkylene-,
      -(C₂-C₄)alkylene-W-X-(C₀-C₃)alkylene-,
      -(C₀-C₄)alkylene-X-W-(C₁-C₃)alkylene-,
      -(C₂-C₅)alkylene-W-X-W-(C₁-C₃)alkylene-, wherein the two occurrences of W are independent of each other,
      -(C₁-C₄)alkylene-ethenylene-(C₁-C₄)alkylene-,
      -(C₁-C₄)alkylene-ethenylene-(C₀-C₂)alkylene-X-(C₀-C₅)alkylene-,
      -(C₁-C₄)alkylene-ethenylene-(C₀-C₂)alkylene-X-W-(C₁-C₃)alkylene-,
      -(C₁-C₄)alkylene-ethynylene-(C₁-C₄)alkylene-, or
      -(C₁-C₄)alkylene-ethynylene-X-(C₀-C₃)alkylene-;
      W is oxy, thio, sulfino, sulfonyl, aminosulfonyl-, -mono-N-(C,-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
   X is a five- or six-membered aromatic ring optionally having one or two heteroatoms selected independently from oxygen, nitrogen, and sulfur; said ring optionally mono-, or di-substituted independently with halo, (C₁-C₃)alkyl, trifluoromethyl, trifluoromethyloxy, difluoromethyloxy, hydroxyl, (C₁-C₄)alkoxy, or carbamoyl;
   Z is carboxyl, (C₁-C₆)alkoxycarbonyl, tetrazolyl, 1,2,4-oxadiazolyl, 5-oxo-1,2,4-oxadiazolyl, (C₁-C₄)alkylsulfonylcarbamoyl or phenylsulfonylcarbamoyl;
   K is a bond, (C₁-C₈)alkylene, thio(C₁-C₄)alkylene or oxy(C₁-C₄)alkylene, said (C₁-C₈)alkylene optionally mono-unsaturated and wherein K is optionally mono-, di- or tri-substituted independently with fluoro, methyl or chloro;
   M is -Ar, -Ar¹-V-Ar², -Ar¹-S-Ar² or -Ar¹-O-Ar² wherein Ar, Ar¹ and Ar² are each independently a partially saturated, fully saturated or fully unsaturated five- to eight-membered ring optionally having one to four heteroatoms selected independently from oxygen, sulfur and nitrogen, or, a bicyclic ring consisting of two fused partially saturated, fully saturated or fully unsaturated five- or six-membered rings, taken independently, optionally having one to four heteroatoms selected independently from nitrogen, sulfur and oxygen;
   said Ar, Ar¹ and Ar² moieties optionally substituted, on one ring if the moiety is monocyclic, or one or both rings if the moiety is bicyclic, on carbon with up to three substituents independently selected from R¹, R² and R³ wherein R¹, R² and R³ are hydroxy, nitro, halo, (C₁-C₆)alkoxy, (C₁-C₄)alkoxy(C₁-C₄)alkyl, (C₁-C₄)alkoxycarbonyl, (C₁-C₇)alkyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   R¹, R² and R³ are optionally mono-, di- or tri-substituted on carbon independently with halo or hydroxy; and
   V is a bond or (C₁-C₃)alkylene optionally mono- or di-substituted independently with hydroxy or fluoro;
(ii) compounds of Formula II 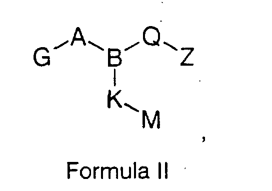 prodrugs thereof, and the pharmaceutically acceptable salts of the compounds and the prodrugs, wherein:
   A is SO₂ or CO;
   G is Ar, Ar¹-V-Ar², Ar-(C₁-C₆)alkylene, Ar-CONH-(C₁-C₆)alkylene, R¹R²-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹ and R² may be taken separately and are independently selected from H and (C₁-C₈)alkyl, or R¹ and R² are taken together with the nitrogen atom of the amino group to form a five- or six-membered azacycloalkyl, said azacycloalkyl optionally containing an oxygen atom and optionally mono-, di- or tri-substituted independently with up to two oxo, hydroxy, (C₁-C₄)alkyl, fluoro or chloro;
   B is N or CH;
   Q is
      -(C₂-C₆)alkylene-W-(C₁-C₃)alkylene-, said alkylenes each optionally substituted with up to four substituents independently selected from fluoro or (C₁-C₄)alkyl,
      -(C₄-C₈)alkylene-, said alkylene optionally substituted with up to four substituents independently selected from fluoro or (C₁-C₄)alkyl,
      -X-(C₁-C₅)alkylene-, said alkylene optionally substituted with up to four substituents independently selected from fluoro or (C₁-C₄)alkyl,
      -(C₁-C₅)alkylene-X-, said alkylene optionally substituted with up to four substituents independently selected from fluoro or (C₁-C₄)alkyl,
      -(C₁-C₃)alkylene-X-(C₁-C₃)alkylene-, said alkylenes each optionally substituted with up to four substituents independently selected from fluoro or (C₁-C₄)alkyl,
      -(C₂-C₄)alkylene-W-X-(C₀-C₃)alkylene-, said alkylenes each optionally substituted with up to four substituents each independently selected from fluoro or (C₁-C₄)alkyl,
      -(C₀-C₄)alkylene-X-W-(C₁-C₃)alkylene-, said alkylenes each optionally substituted with up to four substituents each independently selected from fluoro or (C₁-C₄)alkyl,
      -(C₂-C₅)alkylene-W-X-W-(C₁-C₃)alkylene-, wherein the two occurrences of W are independent of each other, said alkylenes each optionally substituted with up to four substituents each independently selected from fluoro or (C₁-C₄)alkyl,
      -(C₁-C₄)alkylene-ethenylene-(C₁-C₄)alkylene-, said alkylenes and said ethenylene each optionally substituted with up to four substituents each independently selected from fluoro or (C₁-C₄)alkyl,
      -(C₁-C₄)alkylene-ethenylene-(C₀-C₂)alkylene-X-(C₀-C₅)alkylene-, said alkylenes and said ethenylene each optionally substituted with up to four substituents each independently selected from fluoro or (C₁-C₄)alkyl,
      -(C₁-C₄)alkylene-ethenylene-(C₀-C₂)alkylene-X-W-(C₁-C₃)alkylene-, said alkylenes and said ethenylene each optionally substituted with up to four substituents each independently selected from fluoro or (C₁-C₄)alkyl,
      -(C₁-C₄)alkylene-ethynylene-(C₁-C₄)alkylene-, said alkylenes and said ethynylene each optionally substituted with up to four substituents each independently selected from fluoro or (C₁-C₄)alkyl, or
      -(C₁-C₄)alkylene-ethynylene-X-(C₀-C₃)alkylene-, said alkylenes and said ethynylene each optionally substituted with up to four substituents each independently selected from fluoro or (C₁-C₄)alkyl;
      Z is carboxyl, (C₁-C₆)alkoxycarbonyl, tetrazolyl, 1,2,4-oxadiazolyl, 5-oxo-1,2,4-oxadiazolyl, 5-oxo-1,2,4-thiadiazolyl, (C₁-C₄)alkylsulfonylcarbamoyl or phenylsulfonylcarbamoyl;
      K is a bond, (C₁-C₉)alkylene, thio(C₁-C₄)alkylene, (C₁-C₄)alkylenethio(C₁-C₄)alkylene, (C₁-C₄)alkyleneoxy(C₁-C₄)alkylene or oxy(C₁-C₄)alkylene, said (C₁-C₉)alkylene optionally mono-unsaturated and wherein, when K is not a bond, K is optionally mono-, di- or tri-substituted independently with chloro, fluoro, hydroxy or methyl;
      M is -Ar³, -Ar⁴-V¹-Ar⁵, -Ar⁴-S-Ar⁵, -Ar⁴-SO-Ar⁵, -Ar⁴-SO₂-Ar⁵ or -Ar⁴-O-Ar⁵;
   Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five-to seven-membered ring having one or two heteroatoms selected independently from oxygen, sulfur and nitrogen;
   Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³, R⁴ and R⁵ wherein R³, R⁴ and R⁵ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   Ar³, Ar⁴ and Ar⁵ are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar³, Ar⁴ and Ar⁵ moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³¹, R⁴¹ and R⁵¹ wherein R³¹, R⁴¹ and R⁵¹ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
   X is a five- or six-membered aromatic ring optionally having one or two heteroatoms selected independently from oxygen, nitrogen, and sulfur; said ring optionally mono-, di- or tri-substituted independently with halo, (C₁-C₃)alkyl, trifluoromethyl, trifluoromethoxy, difluoromethoxy, hydroxyl, (C₁-C₄)alkoxy, or carbamoyl;
   R¹, R², R³, R⁴ R⁵, R¹¹, R³¹, R⁴¹ and R⁵¹, when containing an alkyl, alkylene, alkenylene or alkynylene moiety, are optionally mono-, di- or tri-substituted on carbon independently with halo or hydroxy; and
   V and V¹ are each independently a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted independently with hydroxy or fluoro;
(iii) compounds of Formula III 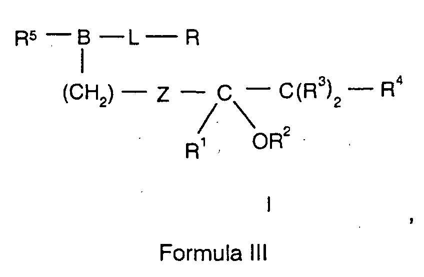 prodrugs thereof, and the pharmaceutically acceptable salts of the compounds and the prodrugs, wherein:
   B is N or C(Q¹), where Q¹ is H or (C₁-C₃)alkyl;
   L is n-propylenyl-X- or CH₂-metaphenylene-CH₂, wherein X is furanyl, thienyl, thiazolyl or tetrahydrofuranyl, said CH₂-metaphenylene-CH₂ or X being optionally mono-, di- or tri-substituted on aromatic carbon independently with one to three chloro, fluoro, methoxy, difluoromethoxy, trifluoromethoxy, trifluoromethyl or methyl;
   R is carboxyl, (C₁-C₆)alkoxycarbonyl, tetrazolyl, 5-oxo-1,2,4-thiadiazolyl; 5-oxo-1,2,4-oxadiazolyl, (C₁-C₄)alkylsulfonylcarbamoyl or phenylsulfonylcarbamoyl;
   R¹ is H, methyl, ethyl or propyl;
   R² is H or (C₂ - C₅) alkanoyl;
   R³ is independently H, fluoro or methyl;
   R⁴ is H, (C₁ - C₇) alkyl, or R⁴ and R¹ are taken together to form a 5-9 membered carbocyclic ring, said alkyl being optionally monounsaturated and optionally mono-, di- or tri-substituted independently with one to three fluoro, chloro, methoxy, difluoromethoxy, trifluoromethoxy, trifluoromethyl or methyl;
   R⁵ is (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl, G-sulfonyl or G-carbonyl, said (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl optionally mono-, di- or tri- substituted on carbon independently with hydroxy, fluoro, chloro, methoxy, difluoromethoxy, trifluoromethoxy, trifluoromethyl or methyl;
   Z is methylene, ethylene, propylene or ethenylene;
   G is Ar, Ar¹-V-Ar², Ar-(C₁-C₆)alkylene, Ar-CONH-(C₁-C₆)alkylene, R¹²R¹³-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹² and R¹³ may be taken separately and are independently selected from H and (C₁-C₈)alkyl, or R¹² and R¹³ are taken together with the nitrogen atom to which they are attached to form a five- or six-membered azacycloalkyl, said azacycloalkyl optionally containing an oxygen atom and optionally substituted with up to two oxo, hydroxy, (C₁-C₄)alkyl, fluoro or chloro;
   Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five to seven-membered ring having one or two heteroatoms selected independently from oxygen, sulfur and nitrogen;
   Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
   said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety, independently selected from R¹⁴, R¹⁵ and R¹⁶ wherein R¹⁴, R¹⁵ and R¹⁶ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl; and
   V is a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted, when V is not a bond, independently with hydroxy or fluoro; and
(iv) compounds of Formula IV 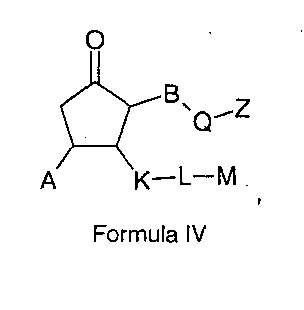 prodrugs thereof, and the pharmaceutically acceptable salts of the compounds and the prodrugs wherein:
   A is hydrogen or hydroxy;
   B is propylene, propenylene or propynylene;
   Q is propylene, -CH₂OCH₂-, thiazolyl, pyridyl, phenyl or thienyl;
   Z is carboxyl, (C₁-C₆)alkoxycarbonyl, tetrazolyl, 1,2,4-oxadiazolyl or 5-oxo-1,2,4-oxadiazolyl;
   K is ethylene or ethenylene;
   L is a bond or -CO-;
   M is -Ar, -Ar¹-V-Ar², -Ar¹-S-Ar² or -Ar¹-O-Ar² wherein
   Ar and Ar¹ are either
      (1) each independently a fully unsaturated five- to eight-membered ring optionally having one to four heteroatoms selected independently from oxygen, sulfur and nitrogen, or a bicyclic ring consisting of two fused partially- saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully-saturated rings optionally having one or more oxo groups substituted on carbon, or
      (2) each independently a fully saturated five to eight membered ring;
   Ar² is a partially saturated, fully saturated or fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon;
   said Ar and Ar¹ moieties, when a fully unsaturated five- to eight-membered ring, a bicyclic ring or a tricyclic ring, and said Ar² moieties are each independently optionally substituted on carbon, on one ring if the moiety is monocyclic, on one or both rings if the moiety is bicyclic, or on one, two or three rings if the moiety is tricyclic, with up to three substituents selected from R¹, R² and R³ wherein R¹, R² and R³ are independently hydroxy, nitro, halo, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, aminocarbonylamino or mono-N-, di-N,N-, di-N,N'- or tri-N,N,N'-(C₁-C₄)alkyl substituted aminocarbonylamino, (C₁-C₄)alkanoylamino, (C₁-C₄)alkoxycarbonylamino, sulfonamido, hydroxysulfonyl, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
   R¹, R² and R³, when containing an alkyl, alkenyl, alkylene or alkenylene moiety, are optionally straight or branched and are optionally mono-, di- or tri-substituted on carbon independently with halo or hydroxy; and
   V is a bond, -CO- or (C₁-C₃)alkylene optionally mono- or di-substituted independently with hydroxy or fluoro.

A preferred subgroup of Formula I compounds comprises those compounds selected from:
7-[(2'-hydroxymethyl-biphenyl-4-ylmethyl)-methanesulfonyl-amino]-heptanoic acid;
7-{[4-(3-hydroxymethyl-thiophen-2-yl)-benzyl]-methanesulfonyl-amino}-heptanoic acid;
7-[(2'-chloro-biphenyl-4-ylmethyl)-methanesulfonyl-amino]-heptanoic acid;
7-{[4-(1-hydroxy-hexyl)-benzyl]-methanesulfonyl-amino}-heptanoic acid;
7-[(4-butyl-benzyl)-methanesulfonyl-amino]-heptanoic acid;
7-{[5-(1-hydroxy-hexyl)-thiophen-2-ylmethyl]-methanesulfonyl-amino}-heptanoic acid;
(3-{[(4-butyl-benzyl)-methanesufonyl-amino]-methyl}-phenyl)-acetic acid;
7-{[3-(3-chloro-phenyl)-propyl]-methanesulfonyl-amino}-heptanoic acid;
7-{[3-(3,5-dichloro-phenyl)-propyl]-methanesufonyl-amino}-heptanoic acid;
5-(3-{[3-(3-chloro-phenyl)-propyl]-methanesulfonyl-amino}-propyl)-thiophene2-carboxylic acid;
7-{[2-(3,5-dichloro-phenoxy)-ethyl]-methanesulfonyl-amino}-heptanoic acid;
5-(3-{[2-(3,5-dichloro-phenoxy)-ethyl]-methanesulfonyl-amino}-propyl)-thiophene-2-carboxylic acid;
N-[2-(3,5-dichloro-phenoxy)-ethyl]-N-[6-(1H-tetrazol-5-yl)-hexyl]-methanesulfonamide;
*trans*-(4-{[3-(3,5-dichloro-phenyl)-allyl]-methanesulfonyl-amino}-butoxy)-acetic acid;
*trans*-N-[3-(3,5-dichloro-phenyl)-allyl]-N-[6-(1H-tetrazol-5-yl)-hexyl]-methanesulfonamide;
*trans*-5-(3-{[3-(3,5-dichloro-phenyl)-allyl]-methanesulfonyl-amino}-propyl)-thiophene-2-carboxylic acid;
*trans*-[3-({[3-(3,5-dichloro-phenyl)-allyl]-methanesulfonyl-amino}-methyl)-phenyl]-acetic acid; the prodrugs thereof, and the pharmaceutically acceptable salts of the compounds, and the prodrugs.

A preferred subgroup of Formula II compounds comprises those compounds selected from:
(3-(((pyridine-3-sulfonyl)-(4-pyrimidin-5-yl-benzyl)-amino)-methyl)-phenyl)-acetic acid;
(3-(((5-phenyl-furan-2-ylmethyl)-(pyridine-3-sulfonyl)-amino)-methyl)-phenyl)-acetic acid;
(3-(((pyridine-3-sulfonyl)-(4-pyrimidin-2-yl-benzyl)-amino)-methyl)-phenyl)-acetic acid;
(3-(((pyridine-3-sulfonyl)-(4-thiazol-2-yl-benzyl)-amino)-methyl)-phenyl)-acetic acid;
(3-(((4-pyrazin-2-yl-benzyl)-(pyridine-3-sulfonyl)-amino)-methyl)-phenyl)-acetic acid;
(3-(((4-cyclohexyl-benzyl)-(pyridine-3-sulfonyl)-amino)-methyl)-phenoxy)-acetic acid;
(3-(((pyridine-3-sulfonyl)-(4-pyridin-2-yl-benzyl)-amino)-methyl)-phenoxy)-acetic acid;
(3-(((pyridine-3-sulfonyl)-(4-pyridin-3-yl-benzyl)-amino)-methyl)-phenoxy)-acetic acid;
(3-(((pyridine-3-sulfonyl)-(4-pyridin-4-yl)-benzyl)-amino)-methyl)-phenoxy)-acetic acid;
(3-(((pyridine-3-sulfonyl)-(4-thiazol-2-yl-benzyl)-amino)-methyl)-phenoxy)-acetic acid;
(3-(((2,3-dihydro-benzo[1,4]dioxin-6-ylmethyl)-(pyridine-3-sulfonyl)-amino)-methyl)-phenyl)-acetic acid;
(3-(((benzofuran-2-ylmethyl-(pyridine-3-sulfonyl)-amino)-methyl)-phenyl)-acetic acid;
(3-(((4-butyl-benzyl)-(pyridine-3-sulfonyl)-amino)-methyl)-phenyl)-acetic acid;
(3-(((benzenesulfonyl-(4-butyl-benzyl)-amino)-methyl)-phenyl)-acetic acid;
(3-(((4-butyl-benzyl)-(1-methyl-1H-imidazole-4-sulfonyl)-amino)-methyl)-phenyl)-acetic acid;
(3-(((4-dimethylamino-benzyl)-(pyridine-3-sulfonyl)-amino)-methyl)-phenyl)-acetic acid;
(3-(((4-dimethylamino-benzyl)-(pyridine-3-sulfonyl)-amino)-methyl)-phenoxy)-acetic acid;
(3-(((4-*tert*-butyl-benzyl)-(pyridine-3-sulfonyl)-amino)-methyl)-phenoxy)-acetic acid;
*trans*-(3-(((3-(3,5-dichloro-phenyl)-allyl)-(pyridine-3-sulfonyl)-amino)-methyl)-phenyl)-acetic acid;
(3-(((2-(3,5-dichloro-phenoxy)-ethyl)-(pyridine-3-sulfonyl)-amino)-methyl)-phenoxy)-acetic acid; the prodrugs thereof, and the pharmaceutically acceptable salts of the compounds, and the prodrugs.

A preferred subgroup of Formula III compounds comprises those compounds wherein:
B is N; R is carboxyl, (C₁-C₆)alkoxycarbonyl or tetrazolyl; Z is ethylenyl; R¹ and R² are each H; and L is CH₂-metaphenylene-CH₂ or n-propylene-X-; the prodrugs thereof, and the pharmaceutically acceptable salts of the compounds, and the prodrugs.
A further preferred subgroup of Formula III compounds comprises those compounds wherein:
R⁵ is selected from (C₁-C₆)alkycarbonyl, optionally mono-, di-, or tri-substituted with hydroxy or fluoro; (C₁-C₃)alkysulfonyl or (C₃-C₇)cycloalkysulfonyl; and G-sulfonyl, wherein G is phenyl, imidazolyl, pyridyl, pyrazolyl, or pyrimidyl optionally mono-, di-, or tri-substituted on carbon or nitrogen with chloro, fluoro, methoxy, difluoromethoxy, trifluoromethoxy, trifluoromethyl or methyl; the prodrugs thereof, and the pharmaceutically acceptable salts of the compounds, and the prodrugs.

A preferred subgroup of Formula IV compounds comprises those compounds selected from:
*trans*-7-(2-(2-(3,5-bis-trifluoromethyl-phenyl)-vinyl)-5-oxo-cyclopentyl)-heptanoic acid;
*trans*-7-(2-(2-(4-chloro-3-trifluoromethyl-phenyl)-vinyl)-5-oxo-cyclcopentyl)-heptanoic acid;
*trans*-7-(2-(2-(3,5-dichlorophenyl)-vinyl-5-oxo-cyclopentyl)-heptanoic acid;
*trans*-7-(2-(2-(3-chlorophenyl-vinyl)-5-oxo-cyclopentyl)-heptanoic acid;
*trans*-7-(2-oxo-5-(2-(3-trifluoromethyl-phenyl)-vinyl)-cyclopentyl)-heptanoic acid;
*trans*-7-(2-(2-(4-fluoro-phenyl)-vinyl)-5-oxo-cyclopentyl)-heptanoic acid;
ethyl *trans*-7-(2-(2-(3,5-bis-trifluoromethyl-phenyl)-vinyl)-5-oxocyclopentyl)-heptanoate;
ethyl *trans*-7-(2-(2-(4-chloro-3-trifluoromethyl-phenyl)-vinyl)-5-oxo-cyclopentyl)-heptanoate;
ethyl *trans*-7-(2-(2-(3,5-dichlorophenyl)-vinyl)-5-oxo-cyclopentyl)-heptanoate;
ethyl *trans*-7-(2-(2-(3-chlorophenyl)-vinyl)-5-oxo-cyclopentyl)-heptanoate;
ethyl *trans*-7-(2-oxo-5-(2-(3-trifluoromethyl-phenyl)-vinyl)-cyclopentyl)-heptanoate;
ethyl *trans*-7-(2-(2-(4-fluoro-phenyl)-vinyl)-5-oxo-cyclopentyl)-heptanoate;
*trans*-3-(2-(3,5-bis-trifluoromethyl-phenyl)-vinyl)-2-(6-(2H-tetrazol-5-yl)-hexyl)-cyclopentanone;
*trans*-3-(2-(4-chloro-3-trifluoromethylphenyl)-vinyl)-2-(6-(2H-tetrazol-5-yl)-hexyl)-cyclopentanone;
*trans*-3-(2-(3,5-dichloro-phenyl)-vinyl)-2-(6-(2H-tetrazol-5-yl)-hexyl)-cyclopentanone;
*trans*-3-(2-(3-chloro-phenyl)-vinyl)-2-(6-(2H-tetrazol-5-yl)-hexyl)-cyclopentanone;
*trans*-3-(2-(3-trifluoromethyl-phenyl)-vinyl)-2-(6-(2H-tetrazol-5-yl)-hexyl)-cyclopentanone;
*trans*-3-(2-(4-fluoro-phenyl)-vinyl)-2-(6-(2H-tetrazol-5-yl)-hexyl)-cyclopentanone; the prodrugs thereof, and the pharmaceutically acceptable salts of the compounds, and the prodrugs.

The compounds of Formula I, the prodrugs thereof, and the pharmaceutically acceptable salts of the compounds and the prodrugs, may be prepared according to the synthetic methodologies described in International Patent Application Publication No. WO 98/28264.

The compounds of Formula II, the prodrugs thereof, and the pharmaceutically acceptable salts of the compounds and the prodrugs, may be prepared according to the synthetic methodologies described in International Application Patent Publication No. WO 99/19300.

The compounds of Formula III, the prodrugs thereof, and the pharmaceutically acceptable salts of the compounds and the prodrugs, may be prepared according to the synthetic methodologies described in European Patent Application Publication No. EP 0 911 321.

The compounds of Formula IV, the prodrugs thereof, and the pharmaceutically acceptable salts of the compounds and the prodrugs, may be prepared according to the synthetic methodologies described in International Patent Application Publication No. WO 98/58911.

Other EP₂ receptor selective agonists which may be used in the compositions, methods and kits of this invention include compounds of the formula 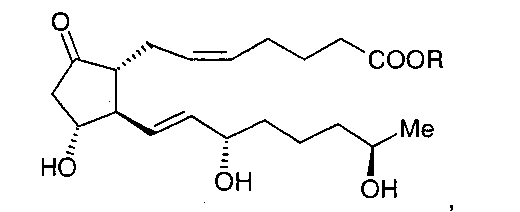 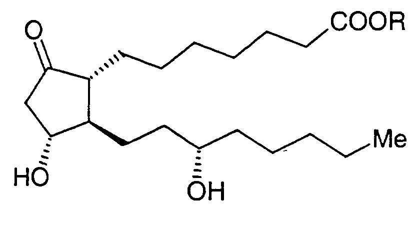 and 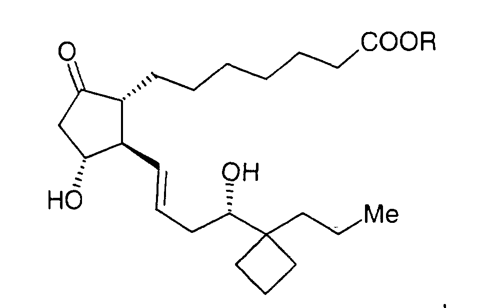 wherein the R is defined, and the compounds are prepared, as disclosed in U.S. Patent No. 5,698,598, which is incorporated herein by reference.

Yet other EP₂ receptor selective agonists which may be used in the compositions, methods and kits of this invention include compounds of the formula 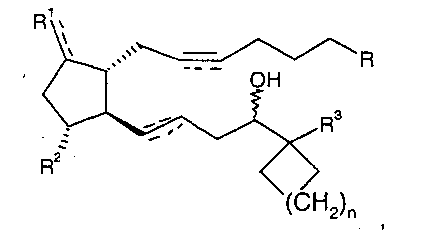 wherein the various substituents are defined, and the compounds are prepared, as disclosed in European Patent Application Publication No. EP 0 860 430, which is incorporated herein by reference.

Still other EP₂ receptor selective agonists which may be used in the compositions, methods and kits of this invention include compounds of the formula 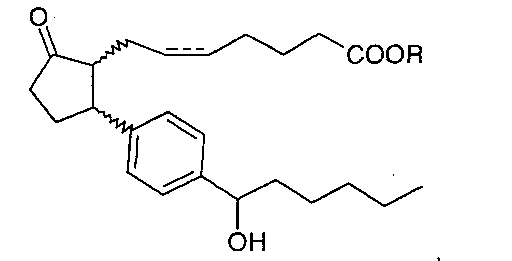 wherein the various substituents are defined, and the compounds are prepared, as disclosed in International Patent Application Publication No. WO95/19964, which is incorporated herein by reference.

Further EP₂ receptor selective agonists which may be used in the compositions, methods and kits of this invention include compounds of the formula 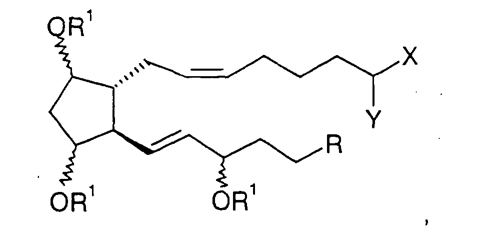 wherein the various substituents are defined, and the compounds are prepared, as disclosed in International Patent Application Publication No. WO99/25358, which is incorporated herein by reference.

Any EP₄ receptor selective agonist may be used as the EP₄ receptor selective agonist of this invention. Preferred EP₄ receptor selective agonists include compounds of Formula V: 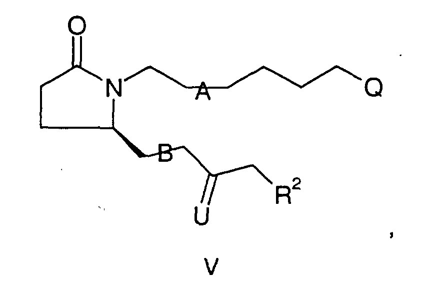 prodrugs thereof and pharmaceutically acceptable salts of said compounds or said prodrugs, wherein:
Q is COOR³, CONHR⁴ or tetrazol-5-yl;
A is a single or cis double bond;
B is a single or trans double bond;
U is 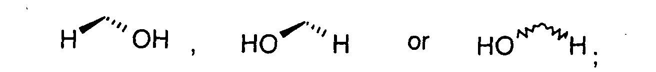
R² is α-thienyl, phenyl, phenoxy, monosubstituted phenyl and monosubstituted phenoxy, said substituents being chloro, fluoro, phenyl, methoxy, trifluoromethyl or (C₁-C₃)alkyl;
R³ is hydrogen, (C₁-C₅)alkyl, phenyl or p-biphenyl;
R⁴ is COR⁵ or SO₂R⁵; and
R⁵ is phenyl or (C₁-C₅)alkyl.

A preferred group of EP₄ receptor selective agonists of Formula V are those compounds of Formula V wherein Q is 5-tetrazolyl. Particularly preferred compounds within this group include 5-(3-hydroxy-4-phenyl-but-1-enyl)-1-[6-(1H-tetrazol-5-yl)-hexyl]-pyrrolidin-2-one and 5-(3-hydroxy-4-phenyl-butyl)-1-[6-(1H-tetrazol-5-yl)-hexyl]-pyrrolidin-2-one.

Another preferred group of EP₄ receptor selective agonists of Formula V are those compound of Formula V wherein Q is COOH. Particularly preferred compounds within this group include 7-(2-(3-hydroxy-4-phenyl-butyl)-5-oxo-pyrrolidin-1-yl)-heptanoic acid and 7-[2-(3-hydroxy-4-phenyl-but-1-enyl)-5-oxo-pyrrolidin-1-yl]-heptanoic acid.

Any EP₂/EP₄ receptor selective agonist may be used as the EP₂/EP₄ receptor selective agonist of this invention. Preferred EP₂/EP₄ receptor selective agonists are compounds of the Formula VI: 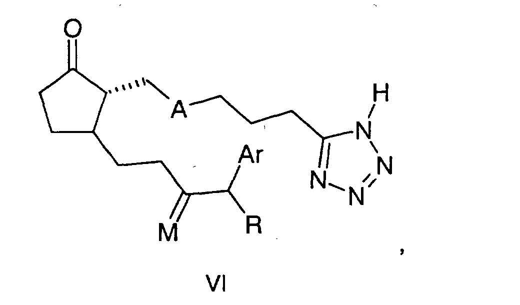 wherein A is ethylene or cis-vinylene; M is 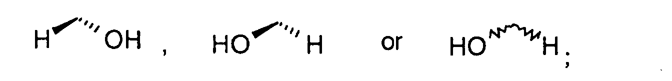 R is hydrogen or methyl; and Ar is phenyl, wherein said phenyl is optionally monosubstituted with fluoro, chloro, bromo, trifluoromethyl, methyl, methoxy or phenyl. Such compounds are prepared as described in U.S. Patent No. 4,097,601, which is incorporated herein by reference. Particularly preferred EP₂/EP₄ receptor selective agonist is the compound of the Formula VI wherein A is ethylene, M is 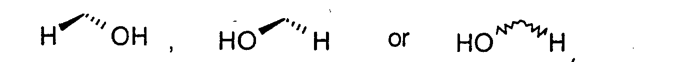 R is hydrogen, and Ar is phenyl, i.e., 3-(3-hydroxy-4-phenyl-butyl)-2-[6-(1 H-tetrazol-5-yl)-hexyl]-cyclopentanone.

The compositions of this invention are all adapted to therapeutic use as agents that stimulate bone formation and increase bone mass in vertebrates, e.g., mammals, and particularly humans. Since bone formation is closely related to the development of osteoporosis and bone related disorders, these compositions, by virtue of their action on bone, prevent, arrest and/or regress osteoporosis.

The utility of the compositions of the present invention as medical agents in the treatment of conditions which present with low bone mass (e.g., osteoporosis) in vertebrates, e.g., mammals (especially humans and particularly female humans) is demonstrated by the activity of the compositions of this invention in conventional assays, including a receptor binding assay, a cyclic AMP assay, an *in vivo* assay and a fracture healing assay (all of which are described below). Such assays also provide a means whereby the activities of the compositions of this invention can be compared to each other and with the activities of other known compounds and compositions. The results of these comparisons are useful for determining dosage levels in a vertebrates, e.g., mammals, including humans, for the treatment of such diseases.

### Anabolic Agent In Vivo Assay

The activity of anabolic bone agents in stimulating bone formation and increasing bone mass can be tested in intact male or female rats, sex hormone deficient male (orchidectomy) or female (ovariectomy) rats.

Male or female rats at different ages (such as 3 months of age) can be used in the study. The rats are either intact or castrated (ovariectomized or orchidectomized), and subcutaneously injected or gavaged with prostaglandin agonists at different doses (such as 1, 3, or 10 mg/kg/day) for 30 days. In the castrated rats, treatment is started at the next day after surgery (for the purpose of preventing bone loss) or at the time bone loss has already occured (for the purpose of restoring bone mass). During the study, all rats are allowed free access to water and a pelleted commercial diet (Teklad Rodent Diet #8064, Harlan Teklad, Madison, WI) containing 1.46% calcium, 0.99% phosphorus and 4.96 IU/g of Vitamin D₃. All rats are given subcutaneous injections of 10 mg/kg calcein on days 12 and 2 before sacrifice. The rats are sacrificed. The following endpoints are determined:

### Femoral Bone Mineral Measurements:

The right femur from each rat is removed at autopsy and scanned using dual energy X-ray absorptiometry (DXA, QDR 1000/W, Hologic Inc., Waltham, MA) equipped with "Regional High Resolution Scan" software (Hologic Inc., Waltham, MA). The scan field size is 5.08 x 1.902 cm, resolution is 0.0254 x 0.0127 cm and scan speed is 7.25 mm/second. The femoral scan images are analyzed and bone area, bone mineral content (BMC), and bone mineral density (BMD) of whole femora (WF), distal femoral metaphyses (DFM), femoral shaft (FS), and proximal femora (PF) are determined.

### Tibial Bone Histomorphometric Analyses:

The right tibia is removed at autopsy, dissected free of muscle, and cut into three parts. The proximal tibia and the tibial shaft are fixed in 70% ethanol, dehydrated in graded concentrations of ethanol, defatted in acetone, then embedded in methyl methacrylate (Eastman Organic Chemicals, Rochester, NY).

Frontal sections of proximal tibial metaphyses at 4 and 10 *µ*m thickness are cut using a Reichert-Jung Polycut S microtome. The 4 *µ*m sections are stained with modified Masson's Trichrome stain while the 10 *µ*m sections remained unstained. One 4 *µ*m and one 10 *µ*m sections from each rat are used for cancellous bone histomorphometry.

Cross sections of tibial shaft at 10 *µ*m thickness are cut using a Reichert-Jung Polycut S microtome. These sections are used for cortical bone histomorphometric analysis.

Cancellous bone histomorphometry: A Bioquant OS/2 histomorphometry system (R&M Biometrics, Inc., Nashville, TN) is used for the static and dynamic histomorphometric measurements of the secondary spongiosa of the proximal tibial metaphyses between 1.2 and 3.6 mm distal to the growth plate-epiphyseal junction. The first 1.2 mm of the tibial metaphyseal region needs to be omitted in order to restrict measurements to the secondary spongiosa. The 4 *µ* m sections are used to determine indices related to bone volume, bone structure, and bone resorption, while the 10 *µ*m sections are used to determine indices related to bone formation and bone turnover.

I) Measurements and calculations related to trabecular bone volume and structure: (1) Total metaphyseal area (TV, mm²): metaphyseal area between 1.2 and 3.6 mm distal to the growth plate-epiphyseal junction. (2) Trabecular bone area (BV, mm²): total area of trabeculae within TV. (3) Trabecular bone perimeter (BS, mm): the length of total perimeter of trabeculae. (4) Trabecular bone volume (BV/TV, %): BV / TV x 100. (5) Trabecular bone number (TBN, #/mm): 1.199 / 2 x BS /TV. (6) Trabecular bone thickness (TBT, *µ*m): (2000/1.199) x (BV / BS). (7) Trabecular bone separation (TBS, *µ*m): (2000 x 1.199) x (TV - BV).

II) Measurements and calculations related to bone resorption: (1) Osteoclast number (OCN, #): total number of osteoclast within total metaphyseal area. (2) Osteoclast perimeter (OCP, mm): length of trabecular perimeter covered by osteoclast. (3) Osteoclast number/mm (OCN/mm, #/mm): OCN / BS. (4) Percent osteoclast perimeter (%OCP, %): OCP /BS x 100.

III) Measurements and calculations related to bone formation and turnover: (1) Single-calcein labeled perimeter (SLS, mm): total length of trabecular perimeter labeled with one calcein label. (2) Double-calcein labeled perimeter (DLS, mm): total length of trabecular perimeter labeled with two calcein labels. (3) Inter-labeled width (ILW, *µ*m): average distance between two calcein labels. (4) Percent mineralizing perimeter (PMS, %): (SLS/2 + DLS) /BS x 100. (5) Mineral apposition rate (MAR, *µ*m/day): ILW / label interval. (6) Bone formation rate/surface ref. (BFR/BS, *µ*m²/d/*µ*m): (SLS/2 + DLS) x MAR / BS. (7) Bone turnover rate (BTR, %/y): (SLS/2 + DLS) x MAR / BV x 100.

Cortical bone histomorphometry: A Bioquant OS/2 histomorphometry system (R&M Biometrics, Inc., Nashville, TN) is used for the static and dynamic histomorphometric measurements of tibial shaft cortical bone. Total tissue area, marrow cavity area, periosteal perimeter, endocortical perimeter, single labeled perimeter, double labeled perimeter, and interlabeled width on both periosteal and endocortical surface are measured, and cortical bone area (total tissue area - marrow cavity area), percent cortical bone area (cortical area / total tissue area x 100), percent marrow area (marrow cavity area / total tissue area x 100), periosteal and endocortical percent labeled perimeter [(single labeled perimeter/2+double labeled perimeter) / total perimeter x 100], mineral apposition rate (interlabeled width/intervals), and bone formation rate [mineral apposition rate x [(single labeled perimeter/2+double labeled perimeter) / total perimeter] are calculated.

### Statistics

Statistics can be calculated using StatView 4.0 packages (Abacus Concepts, Inc., Berkeley, CA). The analysis of variance (ANOVA) test followed by Fisher's PLSD (Stat View, Abacus Concepts Inc., 1918 Bonita Ave, Berkeley, CA 94704-1014) are used to compare the differences between groups.

### Determination of cAMP Elevation in 293-S Cell Lines Stably Overexpressing Recombinant Human EP₂ and EP₄ Receptors.

cDNAs representing the complete open reading frames of the human EP₂ and EP₄ receptors are generated by reverse transcriptase polymerase chain reaction using oligonucleotide primers based on published sequences (1, 2) and RNA from primary human kidney cells (EP₂) or primary human lung cells (EP₄) as templates. cDNAs are cloned into the multiple cloning site of pcDNA3 (Invitrogen Corporation, 3985B Sorrento Valley Blvd., San Diego, CA 92121) and used to transfect 293-S human embryonic kidney cells via calcium phosphate co-precipitation. G418-resistant colonies are expanded and tested for specific [³H]PGE₂ binding. Transfectants demonstrating high levels of specific [³H]PGE₂ binding are further characterized by Scatchard analysis to determine Bmax and Kds for PGE₂. The lines selected for compound screening have approximately 338,400 receptors per cell and a Kd = 12 nM for PGE₂ (EP₂), and approximately 256,400 receptors per cell and a Kd = 2.9 nM for PGE₂ (EP₄). Constituitive expression of both receptors in parental 293-S cells is negligible. Cells are maintained in RPMI supplemented with fetal bovine serum (10% final) and G418 (700 ug/ml final).

cAMP responses in the 293-S/EP₂ and 293-S/EP₄ lines are determined by detaching cells from culture flasks in 1 ml of Ca++ and Mg++ deficient PBS via vigorous pounding, adding serum-free RPMI to a final concentration of 1 X 10⁶ cells/ml, and adding 3-isobutyl-1-methylxanthine (IBMX) to a final concentration of 1 mM. One milliliter of cell suspension is immediately aliquoted into individual 2 ml screwcap microcentrifuge and incubated for 10 minutes, uncovered, at 37 °C, 5% CO₂,95% relative humdity. The compound to be tested is then added to cells at 1:100 dilutions such that final DMSO or ethanol concentrations is 1%. Immediately after adding compound, the tubes are covered, mixed by inverting two times, and incubated at 37 °C for 12 minutes. Samples are then lysed by incubation at 100 °C for 10 minutes and immediately cooled on ice for 5 minutes. Cellular debris is pelleted by centrifugation at 1000 X g for 5 minutes, and cleared lysates are transferred to fresh tubes. cAMP concentrations are determined using a commercially available cAMP radioimmunoassay kit RIA (NEK-033, DuPont/NEN Research Products, 549 Albany St., Boston, MA 02118) after diluting cleared lysates 1:10 in cAMP RIA assay buffer (included in kit). Typically, one treats cells with 6-8 concentrations of the compound to be tested in 1 log increments. EC50 calculations are performed on a calculator using linear regression analysis on the linear portion of the dose response curves.

### References

1. Regan, J.W. Bailey, T.J. Pepperl, D.J. Pierce, K.L. Bogardus,A.M. Donello, J.E. Fairbairn, C.E. Kedzie, K.M. Woodward, D.F. and Gil, D.W. 1994 Cloning of a Novel Human Prostaglandin Receptor with Characteristics of the Pharmaclogically Defined EP₂ Subtype. Mol. Pharmacology 46:213-220.
2. Bastien, L., Sawyer, N., Grygorczyk, R., Metters, K., and Adam, M. 1994 Cloning, Functional Expression, and Characterization of the Human Prostaglandin E2 Receptor EP₂ Subtype. J. Biol. Chem. Vol 269, 16:11873-11877.

### Assay for Binding to Prostaglandin E₂ Receptors

Membrane Preparation: All operations are performed at 4 °C. Transfected cells expressing prostaglandin E₂ type 1 receptors (EP₁), type 2 (EP₂), type 3 (EP₃) or type 4 (EP₄) receptors are harvested and suspended to 2 million cells per ml in Buffer A [50 mM Tris-HCI (pH 7.4), 10 mM MgCl₂, 1 mM EDTA, 1 mM Pefabloc peptide, (Boehringer Mannheim Corp., Indianapolis, IN), 10 uM Phosporamidon peptide, (Sigma, St. Louis, MO), 1 uM pepstatin A peptide, (Sigma, St. Louis, MO), 10 uM elastatinal peptide, (Sigma, St. Louis, MO), 100 uM antipain peptide, (Sigma, St. Louis, MO)]. The cells are lysed by sonification with a Branson Sonifier (Model #250, Branson Ultrasonics Corporation, Danbury, CT) in 2 fifteen second bursts. Unlysed cells and debris are removed by centrifugation at 100 x g for 10 min. Membranes are then harvested by centrifugation at 45,000 x g for 30 minutes. Pelleted membranes are resuspended to 3-10 mg protein per ml, protein concentration being determined of the method of Bradford [Bradford, M., Anal. Biochem., 72, 248 (1976)]. Resuspended membranes are then stored frozen at -80 °C until use.

Binding Assay: Frozen membranes prepared as above are thawed and diluted to 1 mg protein per ml in Buffer A above. One volume of membrane preparation is combined with 0.05 volume test compound or buffer and one volume of 3 nM ³H-prostaglandin E₂ (#TRK 431, Amersham, Arlington Heights, IL) in Buffer A. The mixture (205 µL total volume) is incubated for 1 hour at 25°C. The membranes are then recovered by filtration through type GF/C glass fiber filters ( #1205-401, Wallac, Gaithersburg, MD ) using a Tomtec harvester ( Model Mach II/96, Tomtec, Orange, CT). The membranes with bound ³H-prostaglandin E₂ are trapped by the filter, while the buffer and unbound ³H-prostaglandin E₂ pass through the filter into waste. Each sample is then washed 3 times with 3 ml of [50 mM Tris-HCI (pH 7.4), 10 mM MgCl₂, 1 mM EDTA]. The filters are then dried by heating in a microwave oven. To determine the amount of ³H-prostaglandin bound to the membranes, the dried filters are placed into plastic bags with scintillation fluid and counted in a LKB 1205 Betaplate reader (Wallac, Gaithersburg, MD). IC50s are determined from the concentration of test compound required to displace 50% of the specifically bound ³H-prostaglandin E₂.

The full length EP₁ receptor is made as disclosed in Funk et al., Journal of Biological Chemistry, **1993, 268,** 26767-26772. The full length EP₂ receptor is made as disclosed in Regan et al., Molecular Pharmacology, **1994, 46,** 213-220. The full length EP₃ receptor is made as disclosed in Regan et al., British Journal of Pharmacology, **1994, 112,** 377-385. The full length EP₄ receptor is made as disclosed in Bastien, Journal of Biological Chemistry, **1994, 269,** 11873-11877. These full length receptors are used to prepare 293S cells expressing the EP₁, EP₂, EP₃ and EP₄ receptors.

293S cells expressing either the human EP₁, EP₂, EP₃ or EP₄ prostaglandin E₂ receptors are generated according to methods known to those skilled in the art. Typically, PCR (polymerase chain reaction) primers corresponding to the 5' and 3' ends of the published full length receptor are made according to the well known methods disclosed above and are used in an RT-PCR reaction using the total RNA from human kidney (for EP₁), human lung (for EP₂), human lung (for EP₃) or human lymphocytes (for EP₄) as a source. PCR products are cloned by the TA overhang method into pCR2.1 (Invitrogen, Carlsbad, CA) and identity of the cloned receptor is confirmed by DNA sequencing.

293S cells (Mayo, Dept. of Biochemistry, Northwestern Univ.) are transfected with the cloned receptor in pcDNA3 by electroporation. Stable cell lines expressing the receptor are established following selection of transfected cells with G418.

Clonal cell lines expressing the maximal number of receptors are chosen following a whole cell ³H-PGE₂ binding assay using unlabeled PGE₂ as a competitor.

### FRACTURE HEALING ASSAYS ASSAY FOR EFFECTS ON FRACTURE HEALING AFTER SYSTEMIC ADMINISTRATION

Fracture Technique: Sprage-Dawley rats at 3 months of age are anesthetized with Ketamine. A 1 cm incision is made on the anteromedial aspect of the proximal part of the right tibia or femur. The following describes the tibial surgical technique. The incision is carried through to the bone, and a 1 mm hole is drilled 4 mm proximal to the distal aspect of the tibial tuberosity 2 mm medial to the anterior ridge. Intramedullary nailing is performed with a 0.8 mm stainless steel tube (maximum load 36.3 N, maximum stiffness 61.8 N/mm, tested under the same conditions as the bones). No reaming of the medullary canal is performed. A standardized closed fracture is produced 2 mm above the tibiofibular junction by three-point bending using specially designed adjustable forceps with blunt jaws. To minimize soft tissue damage, care is taken not to displace the fracture. The skin is closed with monofilament nylon sutures. The operation is performed under sterile conditions. Radiographs of all fractures are taken immediately after nailing, and rats with fractures outside the specified diaphyseal area or with displaced nails are excluded. The remaining animals are divided randomly into the following groups with 10 - 12 animals per each subgroup per time point for testing the fracture healing. The first group receives daily gavage of vehicle (water: 100% Ethnanol = 95 : 5) at 1 ml/rat, while the others receive daily gavage from 0.01 to 100 mg/kg/day of the compound to be tested (1 ml/rat) for 10, 20, 40 and 80 days.

At 10, 20, 40 and 80 days, 10 - 12 rats from each group are anesthetized with Ketamine and sacrificed by exsanguination. Both tibiofibular bones are removed by dissection and all soft tissue is stripped. Bones from 5 - 6 rats for each group are stored in 70% ethanol for histological analysis, and bones from another 5 - 6 rats for each group are stored in a buffered Ringer's solution (+4°C, pH 7.4) for radiographs and biomechanical testing which is performed.

Histological Analysis: The methods for histologic analysis of fractured bone have been previously published by Mosekilde and Bak (The Effects of Growth Hormone on Fracture Healing in Rats: A Histological Description. Bone, 14:19-27, 1993). Briefly, the fracture side is sawed 8 mm to each side of the fracture line, embedded undecalcified in methymethacrylate, and cut frontal sections on a Reichert-Jung Polycut microtome 8 *µ*m thick. Masson-Trichrome stained mid-frontal sections (including both tibia and fibula) are used for visualization of the cellullar and tissue response to fracture healing with and without treatment. Sirius red stained sections are used to demonstrate the characterisitics of the callus structure and to differentiate between woven bone and lamellar bone at the fracture site. The following measurements are performed: (1) fracture gap - measured as the shortest distance between the cortical bone ends in the fracture, (2) callus length and callus diameter, (3) total bone volume area of callus, (4) bony tissue per tissue area inside the callus area, (5) fibrous tissue in the callus, and (6) cartilage area in the callus.

Biomechanical Analysis: The methods for biomechanical analysis have been previously published by Bak and Andreassen (The Effects of Aging on Fracture Healing in Rats. Calcif Tissue Int 45:292-297, 1989). Briefly, radiographs of all fractures are taken prior to the biomechanical test. The mechanical properties of the healing fractures are analyzed by a destructive three- or four-point bending procedure. Maximum load, stiffness, energy at maximum load, deflection at maximum load, and maximum stress are determined.

### ASSAY FOR EFFECTS ON FRACTURE HEALING AFTER LOCAL ADMINISTRATION

Fracture Technique: Female or male beagle dogs at approximately 2 years of age are used under anesthesia in the study. Transverse radial fractures are produced by slow continuous loading in three-point bending as described by Lenehan et al. (Lenehan, T. M.; Balligand, M.; Nunamaker, D.M.; Wood, F.E.: Effects of EHDP on Fracture Healing in Dogs. J Orthop Res 3:499-507; 1985). The wire is pulled through the fracture site to ensure complete anatomical disruption of the bone. Thereafter, local delivery of prostaglandin agonists to the fracture site is achieved by slow release of compound delivered by slow release pellets or by administration of the compounds in a suitable Formulation such as a paste gel solution or suspension for 10, 15, or 20 weeks.

Histological Analysis: The methods for histologic analysis of fractured bone have been previously published by Peter et al. (Peter, C.P.; Cook, W.O.; Nunamaker, D.M.; Provost, M. T.; Seedor, J.G.; Rodan, G.A. Effects of alendronate on fracture healing and bone remodeling in dogs. J. Orthop. Res. 14:74-70, 1996) and Mosekilde and Bak (The Effects of Growth Hormone on Fracture Healing in Rats: A Histological Description. Bone, 14:19-27, 1993). Briefly, after sacrifice, the fracture side is sawed 3 cm to each side of the fracture line, embedded undecalcified in methymethacrylate, and cut on a Reichert-Jung Polycut microtome in 8 *µ*m thick frontal sections. Masson-Trichrome stained mid-frontal sections (including both tibia and fibula) are used for visualization of the cellullar and tissue response to fracture healing with and without treatment. Sirius red stained sections are used to demonstrate the characterisitics of the callus structure and to differentiate between woven bone and lamellar bone at the fracture site. The following measurements are performed: (1) fracture gap - measured as the shortest distance between the cortical bone ends in the fracture, (2) callus length and callus diameter, (3) total bone volume area of callus, (4) bony tissue per tissue area inside the callus area, (5) fibrous tissue in the callus, (6) cartilage area in the callus.

Biomechanical Analysis: The methods for biomechanical analysis have been previously published by Bak and Andreassen (The Effects of Aging on Fracture Healing in Rats. Calcif Tissue Int 45:292-297, 1989) and Peter et al. (Peter, C.P.; Cook, W.O.; Nunamaker, D.M.; Provost, M. T.; Seedor, J.G.; Rodan, G.A. Effects of Alendronate On Fracture Healing And Bone Remodeling In Dogs. J. Orthop. Res. 14:74-70, 1996). Briefly, radiographs of all fractures are taken prior to the biomechanical test. The mechanical properties of the healing fractures are analyzed by a destructive three- or four-point bending procedures. Maximum load, stiffness, energy at maximum load, deflection at maximum load, and maximum stress are determined.

### COMBINATION AND SEQUENTIAL TREATMENT PROTOCOL

The following protocols can of course be varied by those skilled in the art. For example, intact male or female rats, sex hormone deficient male (orchidectomy) or female (ovariectomy) rats may be used. In addition, male or female rats at different ages (such as 12 months of age) can be used in the studies. The rats can be either intact or castrated (ovariectomized or orchidectomized), and administered to with anabolic agents such as the compounds of this invention at different doses (such as 1, 3 or 6 mg/kg/day) for a certain period (such as two weeks to two months), and followed by administration of an anti-resorptive agent such as droloxifene at different doses (such as 1,5,10 mg/kg/day) for a certain period (such as two weeks to two months), or a combination treatment with both anabolic agent and anti-resorptive agent at different doses for a certain period (such as two weeks to two months). In the castrated rats, treatment can be started on the next day after surgery (for the purpose of preventing bone loss) or at the time bone loss has already occurred (for the purpose of restoring bone mass).

The rats are sacrificed under ketamine anesthesia. The following endpoints are determined:

Femoral bone mineral measurements are performed as described above in the estrogen agonist/antagonist protocol.

Lumbar Vertebral Bone Mineral Measurements: Dual energy X-ray absorptiometry (QDR 1000/W, Hologic, Inc., Waltham, MA) equipped with a "Regional High Resolution Scan" software (Hologic, Inc., Waltham, MA) is used to determined the bone area, bone mineral content (BMC), and bone mineral density (BMD) of whole lumbar spine and each of the six lumbar vertebrae (LV1 - 6) in the anesthetized rats. The rats are anesthetized by injection (i.p.) of 1 ml/kg of a mixture of ketamine/rompun (ratio of 4 to 3), and then placed on a rat platform. The scan field sized is 6 x 1.9 cm, resolution is 0.0254 x 0.0127 cm, and scan speed is 7.25 mm/sec. The whole lumbar spine scan image is obtained and analyzed. Bone area (BA), and bone mineral content (BMC) is determined, and bone mineral density is calculated (MBC divided by BA) for the whole lumbar spine and each of the six lumbar vertebrae (LV1 - 6).

Proximal tibial metaphyseal cancellous bone histomorphometric analyses are performed as described above for in the estrogen agonist/antagonist protocol.

Measurements and calculations related to trabecular bone volume and structure are performed as described above in the estrogen agonist/antagonist protocol. Further, measurements and calculations related to bone resorption are also performed as described above in the estrogen agonist/antagonist protocol. Still further, measurements and calculations related to bone formation and turnover are performed as described above in the estrogen agonist/antagonist protocol. Further still, the data obtained is analyzed using the statistical manipulations described above in the estrogen agonist/antagonist protocol.

Administration of the compositions of this invention or of a combination of an EP₂ receptor selective agonist, a prodrug thereof or a pharmaceutically acceptable salt of said agonist or said prodrug and an EP₄ receptor selective agonist, a prodrug thereof or a pharmaceutically acceptable salt of said agonist or said prodrug can be via any method which delivers the composition of this invention or EP₂ receptor selective agonist and EP₄ receptor selective agonist systemically and/or locally (e.g., at the site of the bone fracture, osteotomy, or orthopedic surgery). These methods include oral routes, parenteral, intraduodenal routes, etc. Generally, the compounds of this invention are administered orally, but parenteral administration (e.g., intravenous, intramuscular, transdermal, subcutaneous, rectal or intramedullar) may be utilized, for example, where oral administration is inappropriate for the target or where the patient is unable to ingest the drug.

The pharmaceutical compositions of this invention and the EP₂ receptor selective agonist and EP₄ receptor selective agonist combinations can be used for the treatment and promotion of healing of bone fractures and osteotomies by the local application (e.g., to the sites of bone fractures of osteotomies) of the compositions of this invention. The compositions of this invention and the EP₂ receptor selective agonist and EP₄ receptor selective agonist are applied to the sites of bone fractures or osteotomies, for example, either by injection of the compound in a suitable solvent (e.g., an oily solvent such as arachis oil) to the cartilage growth plate or, in cases of open surgery, by local application thereto of such compositions in a suitable vehicle, carrier or diluent such as bone-wax, demineralized bone powder, polymeric bone cements, bone sealants, etc. Alternatively, local application can be achieved by applying a solution or dispersion of the composition or the EP₂ receptor selective agonist and EP₄ receptor selective agonist combination in a suitable carrier or diluent onto the surface of, or incorporating it into solid or semi-solid implants conventionally used in orthopedic surgery, such as dacron-mesh, gel-foam and kiel bone, or prostheses.

In any event, the amount and timing of compositions administered will, of course, be dependent on the subject being treated, on the severity of the affliction, on the manner of administration and on the judgment of the prescribing physician. Thus, because of patient to patient variability, the dosages given above are a guideline and the physician may titrate doses of the drug compounds to achieve the treatment (e.g., bone mass augmentation) that the physician considers appropriate for the patient. In considering the degree of treatment desired, the physician must balance a variety of factors such as bone mass starting level, age of the patient, presence of preexisting disease, as well as presence of other diseases (e.g., cardiovascular disease).

In general an amount of a composition or combination of an EP₂ receptor selective agonist and EP₄ receptor selecgive agonist of this invention is used that is sufficient to augment bone mass to a level which is above the bone fracture threshold (as detailed in the World Health Organization Study previously cited herein).

The EP₂ receptor selective compounds and EP₄ receptor selective compounds used in the compositions, methods and kits of the present invention are generally administered in the form of a pharmaceutical composition comprising at least one of the compounds of this invention together with a pharmaceutically acceptable vehicle or diluent. Thus, the compounds of this invention can be administered individually or together in any conventional form such as oral, parenteral, rectal or transdermal dosage form.

For oral administration the pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compositions of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see. Remington's Pharmaceutical Sciences, Mack Publishing Company, Easton, Pa., 19th Edition (1995).

Pharmaceutical compositions of the invention may contain a total of 0.1%-95% of an EP₂ receptor selective agonist and of an EP₄ receptor selective agonist used in this invention, preferably 1%-70%. In any event, the composition or formulation to be administered will contain a quantity of the EP₄ receptor selective agonist and the EP₂ receptor selective agonist in an amount effective to treat the disease/condition of the subject being treated, e.g., a bone disorder.

Since the present invention has an aspect that relates to the augmentation and maintenance of bone mass by treatment with a combination of active ingredients which may be administered separately, the invention also relates to combining separate pharmaceutical compositions in kit form. The kit comprises two separate pharmaceutical compositions: an EP₂ receptor selective compound, a prodrug thereof or a pharmaceutically acceptable salt of said EP₂ receptor selective compound or of said prodrug, and an EP₄ receptor selective compound, a prodrug thereof or a pharmaceutically acceptable salt of said EP₄ receptor selective compound or of said prodrug as described above. The kit comprises a container for containing the separate compositions such as a divided bottle or a divided foil packet, however, the separate compositions may also be contained within a single, undivided container. Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of an EP₂ receptor selective compound, prodrug thereof or pharmaceutically acceptable salt of said EP₂ receptor selective compound or said prodrug can consist of one tablet or capsule while a daily dose of the EP₄ receptor selective compound, prodrug thereof or pharmaceutically acceptable salt of said EP₄ receptor selective compound or of said prodrug can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

### Example One

### Study Protocol

Prostaglandin E2 (PGE2) restores bone mass by stimulating both bone formation and bone resorption but in favor of bone formation in ovariectomized (OVX) rat skeleton. The purpose of this study was to determine the skeletal effects of 3-(3-hydroxy-4-phenyl-butyl)-2-(6-(1 H-tetrazol-5-yl)-hexyl)-cyclopentanone, an EP₂ and EP₄ prostaglandin receptor selective agonist (IC50 for EP₂ and EP₄ receptor binding equals 56 and 70 nM, respectively. IC50 for EP₁, EP₃, DP, IP and FP receptor binding are all >3200 nM) in osteopenic, OVX rats.

Sprague-Dawley female rats were sham-operated (n=20) or OVX (n=50) at 3 months of age. Five weeks post-surgery, OVX rats were treated (subcutaneous injection) with either vehicle or 3-(3-hydroxy-4-phenyl-butyl)-2-(6-(1H-tetrazol-5-yl)-hexyl)-cyclopentanone at 1C mg/kg/day for 4 weeks. Distal femoral metaphyseal bone mineral content (BMC) and bone mineral density (BMD) were determined by dual energy x-ray absorptiometry (Hologic QDR-1000/W, Hologic Inc., Waltham, MA) according to the methods described by Ke et al., (Droloxifene, a New Estrogen Antagonist/Agonist, Prevents Bone Loss in Ovariectomized Rats, Endocrinology, 136:2435-2441; 1995).

### Study Results and Discussion

OVX induced significant decrease in BMC (-15%) and BMD (-17%) at 5 weeks post-surgery as compared with sham-operated controls. Continuous decreases in BMC and BMD were seen between 5 and 9 weeks post-surgery in OVX rats (-23% in both BMC and BMD). 3-(3-hydroxy-4-phenyl-butyl)-2-(6-(1H-tetrazol-5-yl)-hexyl)-cyclopentanone at 10 mg/kg/day significantly increased BMC and BMD as compared with pre-treatment OVX controls (+23% and +17% for BMC and BMD, respectively) and OVX controls (+42% and +28% for BMC and BMD, respectively).

These data showed that 3-(3-hydroxy-4-phenyl-butyl)-2-(6-(1 H-tetrazol-5-yl)-hexyl)-cyclopentanone, an EP₂/EP₄ receptor selective agonist, completely restored the bone mass to the OVX, osteopenic rat skeleton. These results indicated EP₂/EP₄ receptor selective agonists are useful agents in treatment of osteoporosis.

### Example Two

### Study Protocol

Prostaglandin E2 (PGE2) restores bone mass by stimulating both bone formation and bone resorption but in favor of bone formation in ovariectomized (OVX) rat skeleton. The purpose of this study was to determine the skeletal effects of combination of 7-(2-(3,5--dichloro-phenoxy)-ethyl)-methanesulfonyl-amino)-heptanoic acid, an EP₂ prostaglandin receptor selective agonist (IC50 for EP₂ receptor binding equals 17 nM), and 7-(2-(3-hydroxy-4-phenyl-butyl)-5-oxo-pyrrolidin-1-yl)-heptanoic acid, an EP₄ prostaglandin receptor selective agonist (IC50 for EP₄ receptor binding equals 36 nM) in osteopenic, OVX rats.

Sprague-Dawley female rats were sham-operated (n=20) or OVX (n=30) at 3 months of age. Five weeks post-surgery, OVX rats were treated (subcutaneous injection) with either vehicle or combination of 7-(2-(3,5--dichloro-phenoxy)-ethyl)-methanesulfonyl-amino)-heptanoic acid (10 mg/kg/day, s.c. injection) and 7-(2-(3-hydroxy-4-phenyl-butyl)-5-oxo-pyrrolidin-1-yl)-heptanoic acid (10 mg/kg/day, s.c. injection) for 4 weeks. Distal femoral metaphyseal bone mineral content (BMC) and bone mineral density (BMD) were determined by dual energy x-ray absorptiometry (Hologic QDR-1000/W, Hologic Inc., Waltham, MA) according to the methods described by Ke et al., (Droloxifene, a New Estrogen Antagonist/Agonist, Prevents Bone Loss in Ovariectomized Rats, Endocrinology, 136:2435-2441; 1995).

### Study Results and Discussion

OVX induced significant decrease in BMC (-13%) and BMD (-13%) at 5 weeks post-surgery as compared with sham-operated controls. Continuous decreases in BMC and BMD were seen between 5 and 9 weeks post-surgery in OVX rats (-26% in BMC and -24% in BMD). A combination of 7-(2-(3,5--dichloro-phenoxy)-ethyl)-methanesulfonyl-amino)-heptanoic acid (10 mg/kg/day, s.c. injection) and 7-(2-(3-hydroxy-4-phenyl-butyl)-5-oxo-pyrrolidin-1-yl)-heptanoic acid (10 mg/kg/day, s.c. injection) significantly increased BMC and BMD as compared with pre-treatment OVX controls (+14% and +8% for BMC and BMD, respectively) and OVX controls (+16% and +12% for BMC and BMD, respectively).

These data show that treatment with combination of an EP₂ and an EP₄ receptor selective agonists restored bone mass to the OVX, osteopenic rat skeleton. These results demonstrate that these therapeutic regimens are useful in treatment of osteoporosis.

## Claims

1. The use of an EP₂ receptor selective agonist, prodrug or pharmaceutically acceptable salt thereof in the preparation of a medicament in combination with an EP₄ receptor selective agonist, prodrug or a pharmaceutically acceptable salt thereof, for the treatment of a condition which presents with low bone mass in mammals.

2. The use as claimed in claims 1 wherein said EP₂ receptor selective agonist is selected from: 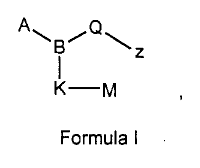 prodrugs thereof, and the pharmaceutically acceptable salts of the compounds and the prodrugs, wherein:
B is N;
A is (C₁-C₆)alkylsulfonyl, (C₃-C₇)cycloalkylsulfonyl, (C₃-C₇)cycloalkyl(C₁-C₆)alkylsulfonyl, said A moieties optionally mono-, di- or tri- substituted on carbon independently with hydroxy, (C₁-C₄)alkyl or halo;
Q is
-(C₂-C₆)alkylene-W-(C₁-C₃)alkylene-,
-(C₃-C₈)alkylene-, said -(C₃-C₈)alkylene- optionally substituted with up to four substituents independently selected from fluoro or (C₁-C₄)alkyl,
-X-(C₁-C₅)alkylene-,
-(C₁-C₅)alkylene-X-,
-(C₁-C₃)alkylene-X-(C₁-C₃)alkylene-,
-(C₂-C₄)alkylene-W-X-(C₀-C₃)alkylene-,
-(C₀-C₄)alkylene-X-W-(C₁-C₃)alkylene-,
-(C₂-C₅)alkylene-W-X-W-(C₁-C₃)alkylene-, wherein the two occurrences of W are independent of each other,
-(C₁-C₄)alkylene-ethenylene-(C₁-C₄)alkylene-,
-(C₁-C₄)alkylene-ethenylene-(C₀-C₂)alkylene-X-(C₀-C₅)alkylene-,
-(C₁-C₄)alkylene-ethenylene-(C₀-C₂)alkylene-X-W-(C₁-C₃)alkylene-,
-(C₁-C₄)alkylene-ethynylene-(C₁-C₄)alkylene-, or
-C₁-C₄)alkylene-ethynylene-X-(C₀-C₃)alkylene-;
W is oxy, thio, sulfino, sulfonyl, aminosufonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
X is a five- or six-membered aromatic ring optionally having one or two heteroatoms selected independently from oxygen, nitrogen, and sulfur; said ring optionally mono-, or di-substituted independently with halo, (C₁-C₃)alkyl, trifluoromethyl, trifluoromethyloxy, difluoromethyloxy, hydroxyl, (C₁-C₄)alkoxy, or carbamoyl;
Z is carboxyl, (C₁-C₆)alkoxycarbonyl, tetrazolyl, 1,2,4-oxadiazolyl, 5-oxo-1,2,4-oxadiazolyl, (C₁-C₄)alkylsulfonylcarbamoyl or phenylsulfonylcarbamoyl;
K is a bond, (C₁-C₈)alkylene, thio(C₁-C₄)alkylene or oxy(C₁-C₄)alkylene, said (C₁-C₈)alkylene optionally mono-unsaturated and wherein K is optionally mono-, di- or tri-substituted independently with fluoro, methyl or chloro;
M is -Ar, -Ar¹-V-Ar², -Ar¹-S-Ar² or -Ar¹-O-Ar² wherein Ar, Ar¹ and Ar² are each independently a partially saturated, fully saturated or fully unsaturated five- to eight-membered ring optionally having one to four heteroatoms selected independently from oxygen, sulfur and nitrogen, or, a bicyclic ring consisting of two fused partially saturated, fully saturated or fully unsaturated five- or six-membered rings, taken independently, optionally having one to four heteroatoms selected independently from nitrogen, sulfur and oxygen;
said Ar, Ar¹ and Ar² moieties optionally substituted, on one ring if the moiety is monocyclic, or one or both rings if the moiety is bicyclic, on carbon with up to three substituents independently selected from R¹, R² and R³ wherein R¹, R² and R³ are hydroxy, nitro, halo, (C₁-C₆)alkoxy, (C₁-C₄)alkoxy(C₁-C₄)alkyl, (C₁-C₄)alkoxycarbonyl, (C₁-C₇)alkyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
R¹, R² and R³ are optionally mono-, di- or tri-substituted on carbon independently with halo or hydroxy; and
V is a bond or (C₁-C₃)alkylene optionally mono- or di-substituted independently with hydroxy or fluoro;
(ii) compounds of Formula II 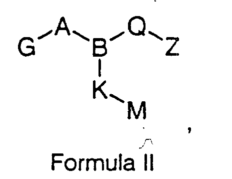 prodrugs thereof, and the pharmaceutically acceptable salts of the compounds and the prodrugs, wherein:
A is SO₂ or CO;
G is Ar, Ar¹-V-Ar², Ar-(C₁-C₆)alkylene, Ar-CONH-(C₁-C₆)alkylene, R¹R²-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹ and R² may be taken separately and are independently selected from H and (C₁-C₈)alkyl, or R¹ and R² are taken together with the nitrogen atom of the amino group to form a five- or six-membered azacycloalkyl, said azacycloalkyl optionally containing an oxygen atom and optionally mono-, di- or tri-substituted independently with up to two oxo, hydroxy, (C₁-C₄)alkyl, fluoro or chloro;
B is N or CH;
Q is
-(C₂-C₆)alkylene-W-(C₁-C₃)alkylene-, said alkylenes each optionally substituted with up to four substituents independently selected from fluoro or (C₁-C₄)alkyl,
-(C₄-C₈)alkylene-, said alkylene optionally substituted with up to four substituents independently selected from fluoro or (C₁-C₄)alkyl,
-X-(C₁-C₅)alkylene-, said alkylene optionally substituted with up to four substituents independently selected from fluoro or (C₁-C₄)alkyl,
-(C₁-C₅)alkylene-X-, said alkylene optionally substituted with up to four substituents independently selected from fluoro or (C₁-C₄)alkyl,
-(C₁-C₃)alkylene-X-(C₁-C₃)alkylene-, said alkylenes each optionally substituted with up to four substituents independently selected from fluoro or (C₁-C₄)alkyl,
-(C₂-C₄)alkylene-W-X-(C₀-C₃)alkylene-, said alkylenes each optionally substituted with up to four substituents each independently selected from fluoro or (C₁-C₄)alkyl,
-(C₀-C₄)alkylene-X-W-(C₁-C₃)alkylene-, said alkylenes each optionally substituted with up to four substituents each independently selected from fluoro or (C₁-C₄)alkyl,
-(C₂-C₅)alkylene-W-X-W-(C₁-C₃)alkylene-, wherein the two occurrences of W are independent of each other, said alkylenes each optionally substituted with up to four substituents each independently selected from fluoro or (C₁-C₄)alkyl,
-(C₁-C₄)alkylene-ethenylene-(C₁-C₄)alkylene-, said alkylenes and said ethenylene each optionally substituted with up to four substituents each independently selected from fluoro or (C₁-C₄)alkyl,
-(C₁-C₄)alkylene-ethenylene-(C₀-C₂)alkylene-X-(C₀-C₅)alkylene-, said alkylenes and said ethenylene each optionally substituted with up to four substituents each independently selected from fluoro or (C₁-C₄)alkyl,
-(C₁-C₄)alkylene-ethenylene-(C₀-C₂)alkylene-X-W-(C₁-C₃)alkylene-, said alkylenes and said ethenylene each optionally substituted with up to four substituents each independently selected from fluoro or (C₁-C₄)alkyl,
-(C₁-C₄)alkylene-ethynylene-(C₁-C₄)alkylene-, said alkylenes and said ethynylene each optionally substituted with up to four substituents each independently selected from fluoro or (C₁-C₄)alkyl, or
-(C₁-C₄)alkylene-ethynylene-X-(C₀-C₃)alkylene-, said alkylenes and said ethynylene each optionally substituted with up to four substituents each independently selected from fluoro or (C₁-C₄)alkyl;
Z is carboxyl, (C₁-C₆)alkoxycarbonyl, tetrazolyl, 1,2,4-oxadiazolyl, 5-oxo-1,2,4-oxadiazolyl, 5-oxo-1,2,4-thiadiazolyl, (C₁-C₄)alkylsulfonylcarbamoyl or phenylsulfonylcarbamoyl;
K is a bond, (C₁-C₉)alkylene, thio(C₁-C₄)alkylene, (C₁-C₄)alkylenethio(C₁-C₄)alkylene, (C₁-C₄)alkyleneoxy(C₁-C₄)alkylene or oxy(C₁-C₄)alkylene, said (C₁-C₉)alkylene optionally mono-unsaturated and wherein, when K is not a bond, K is optionally mono-, di- or tri-substituted independently with chloro, fluoro, hydroxy or methyl;
M is -Ar³, -Ar⁴-V¹-Ar⁵, -Ar⁴-S-Ar⁵, -Ar⁴-SO-Ar⁵, -Ar⁴-SO₂-Ar⁵ or -Ar⁴-O-Ar⁵;
Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five-to seven-membered ring having one or two heteroatoms selected independently from oxygen, sulfur and nitrogen;
Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³, R⁴ and R⁵ wherein R³, R⁴ and R⁵ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
Ar³, Ar⁴ and Ar⁵ are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; said Ar³, Ar⁴ and Ar⁵ moieties are optionally substituted on carbon or nitrogen, on one ring if the moiety is monocyclic, on one or both rings if the moiety is bicyclic, or on one, two or three rings if the moiety is tricyclic, with up to three substituents per moiety independently selected from R³¹, R⁴¹ and R⁵¹ wherein R³¹, R⁴¹ and R⁵¹ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)aikoxycarbonyiamino, hydroxysulfonyl, aminocarbonylamino or mono-N-, di-N,N-, di-N,N'-or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
W is oxy, thio, sulfino, sulfonyl, aminosulfonyl-, -mono-N-(C₁-C₄)alkyleneaminosulfonyl-, sulfonylamino, N-(C₁-C₄)alkylenesulfonylamino, carboxamido, N-(C₁-C₄)alkylenecarboxamido, carboxamidooxy, N-(C₁-C₄)alkylenecarboxamidooxy, carbamoyl, -mono-N-(C₁-C₄)alkylenecarbamoyl, carbamoyloxy, or -mono-N-(C₁-C₄)alkylenecarbamoyloxy, wherein said W alkyl groups are optionally substituted on carbon with one to three fluorines;
X is a five- or six-membered aromatic ring optionally having one or two heteroatoms selected independently from oxygen, nitrogen, and sulfur; said ring optionally mono-, di- or tri-substituted independently with halo, (C₁-C₃)alkyl, trifluoromethyl, trifluoromethoxy, difluoromethoxy, hydroxyl, (C₁-C₄)alkoxy, or carbamoyl;
R¹, R², R³, R⁴ R⁵, R¹¹, R³¹, R⁴¹ and R⁵¹, when containing an alkyl, alkylene, alkenylene or alkynylene moiety, are optionally mono-, di- or tri-substituted on carbon independently with halo or hydroxy; and
V and V¹ are each independently a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted independently with hydroxy or fluoro;
(iii) compounds of Formula III 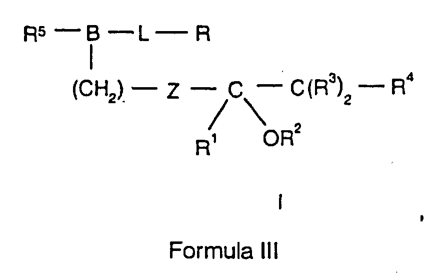 prodrugs thereof, and the pharmaceutically acceptable salts of the compounds and the prodrugs, wherein:
B is N or C(Q¹), where Q¹ is H or (C₁-C₃)alkyl;
L is n-propylenyl-X- or CH₂-metaphenylene-CH₂, wherein X is furanyl, thienyl, thiazolyl or tetrahydrofuranyl, said CH₂-metaphenylene-CH₂ or X being optionally mono-, di- or tri-substituted on aromatic carbon independently with one to three chloro, fluoro, methoxy, difluoromethoxy, trifluoromethoxy, trifluoromethyl or methyl;
R is carboxyl, (C₁-C₆)alkoxycarbonyl, tetrazolyl, 5-oxo-1,2,4-thiadiazolyl; 5-oxo-1,2,4-oxadiazolyl, (C₁-C₄)alkylsulfonylcarbamoyl or phenylsulfonylcarbamoyl;
R¹ is H, methyl, ethyl or propyl;
R² is H or (C₂ - C₅) alkanoyl;
R³ is independently H, fluoro or methyl;
R⁴ is H, (C₁ - C₇) alkyl, or R⁴ and R¹ are taken together to form a 5-9 membered carbocyclic ring, said alkyl being optionally monounsaturated and optionally mono-, di- or tri-substituted independently with one to three fluoro, chloro, methoxy, difluoromethoxy, trifluoromethoxy, trifluoromethyl or methyl;
R⁵ is (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl, G-sulfonyl or G-carbonyl, said (C₁-C₆)alkylsulfonyl, (C₃-C₇)cycloalkylsulfonyl, (C₃-C₇)cycloalkyl(C₁-C₆)alkylsulfonyl, (C₁-C₆)alkylcarbonyl, (C₃-C₇)cycloalkylcarbonyl, (C₃-C₇)cycloalkyl(C₁-C₆)alkylcarbonyl optionally mono-, di- or tri- substituted on carbon independently with hydroxy, fluoro, chloro, methoxy, difluoromethoxy, trifluoromethoxy, trifluoromethyl or methyl;
Z is methylene, ethylene, propylene or ethenylene;
G is Ar, Ar¹-V-Ar², Ar-(C₁-C₆)alkylene, Ar-CONH-(C₁-C₆)alkylene, R¹²R¹³-amino, oxy(C₁-C₆)alkylene, amino substituted with Ar, or amino substituted with Ar(C₁-C₄)alkylene and R¹¹, wherein R¹¹ is H or (C₁-C₈)alkyl, R¹² and R¹³ may be taken separately and are independently selected from H and (C₁-C₈)alkyl, or R¹² and R¹³ are taken together with the nitrogen atom to which they are attached to form a five- or six-membered azacycloalkyl, said azacycloalkyl optionally containing an oxygen atom and optionally substituted with up to two oxo, hydroxy, (C₁-C₄)alkyl, fluoro or chloro;
Ar is a partially saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five- or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur; or Ar is a fully saturated five to seven-membered ring having one or two heteroatoms selected independently from oxygen, sulfur and nitrogen;
Ar¹ and Ar² are each independently a partially saturated, fully saturated or fully unsaturated five- to eight-membered ring optionally having one to four heteroatoms selected independently from oxygen, sulfur and nitrogen, or a bicyclic ring consisting of two fused independently partially saturated, fully saturated or fully unsaturated five-or six-membered rings, taken independently, optionally having one to four heteroatoms selected independently from nitrogen, sulfur and oxygen, or a tricyclic ring consisting of three fused independently partially saturated, fully saturated or fully unsaturated five- or six-membered rings, taken independently, optionally having one to four heteroatoms selected independently from nitrogen, sulfur and oxygen, said partially or fully saturated ring, bicyclic ring or tricyclic ring optionally having one or two oxo groups substituted on carbon or one or two oxo groups substituted on sulfur;
said Ar, Ar¹ and Ar² moieties are optionally substituted on carbon or nitrogen, on one ring if the moiety is monocyclic, on one or both rings if the moiety is bicyclic, or on one, two or three rings if the moiety is tricyclic, with up to three substituents per moiety, independently selected from R¹⁴, R¹⁵ and R¹⁶ wherein R¹⁴, R¹⁵ and R¹⁶ are independently hydroxy, nitro, halo, carboxy, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, (C₁-C₄)alkanoylamino, (C₁-C₄)alkoxycarbonylamino, hydroxysulfonyl, aminocarbonylamino or mono-N-, di-N,N-, di-N,N'- or tri-N,N,N'-(C₁-C₄)alkyl substituted aminocarbonylamino, sulfonamido, (C₁-C₄)alkylsulfonamido, amino, mono-N- or di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl; and
V is a bond, thio(C₁-C₄)alkylene, (C₁-C₄)alkylenethio, (C₁-C₄)alkyleneoxy, oxy(C₁-C₄)alkylene or (C₁-C₃)alkylene optionally mono- or di-substituted, when V is not a bond, independently with hydroxy or fluoro; and
(iv) compounds of Formula IV 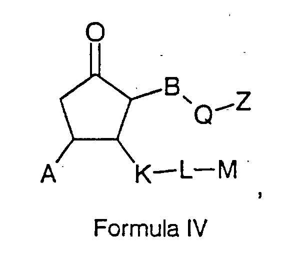 prodrugs thereof, and the pharmaceutically acceptable salts of the compounds and the prodrugs wherein:
A is hydrogen or hydroxy;
B is propylene, propenylene or propynylene;
Q is propylene, -CH₂OCH₂-, thiazolyl, pyridyl, phenyl or thienyl;
Z is carboxyl, (C₁-C₆)alkoxycarbonyl, tetrazolyl, 1,2,4-oxadiazolyl or 5-oxo-1,2,4-oxadiazolyl;
K is ethylene or ethenylene;
L is a bond or -CO-;
M is -Ar, -Ar¹-V-Ar², -Ar¹-S-Ar² or -Ar¹-O-Ar² wherein
Ar and Ar¹ are either
(1) each independently a fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon, or
(2) each independently a fully saturated five to eight membered ring;
Ar² is a partially saturated, fully saturated or fully unsaturated five- to eight-membered ring optionally having one to four heteroatoms selected independently from oxygen, sulfur and nitrogen, or a bicyclic ring consisting of two fused partially saturated, fully saturated or fully unsaturated five- and/or six-membered rings, taken independently, optionally having one to four heteroatoms selected independently from nitrogen, sulfur and oxygen, or a tricyclic ring consisting of three fused partially saturated, fully saturated or fully unsaturated five- and/or six-membered rings, taken independently, optionally having one to four heteroatoms selected independently from nitrogen, sulfur and oxygen, any of said partially saturated or fully saturated rings optionally having one or more oxo groups substituted on carbon;
said Ar and Ar¹ moieties, when a fully unsaturated five- to eight-membered ring, a bicyclic ring or a tricyclic ring, and said Ar² moieties are each independently optionally substituted on carbon, on one ring if the moiety is monocyclic, on one or both rings if the moiety is bicyclic, or on one, two or three rings if the moiety is tricyclic, with up to three substituents selected from R¹, R² and R³ wherein R¹, R² and R³ are independently hydroxy, nitro, halo, (C₁-C₇)alkoxy, (C₁-C₄)alkoxy(C₁-C₄)alkyl, (C₁-C₄)alkoxycarbonyl, (C₁-C₇)alkyl, (C₂-C₇)alkenyl, (C₂-C₇)alkynyl, (C₃-C₇)cycloalkyl, (C₃-C₇)cycloalkyl(C₁-C₄)alkyl, (C₃-C₇)cycloalkyl(C₁-C₄)alkanoyl, formyl, (C₁-C₈)alkanoyl, (C₁-C₆)alkanoyl(C₁-C₆)alkyl, aminocarbonylamino or mono-N-, di-N,N-, di-N,N'- or tri-N,N,N'-(C₁-C₄)alkyl substituted aminocarbonylamino, (C₁-C₄)alkanoylamino, (C₁-C₄)alkoxycarbonylamino, sulfonamido, hydroxysulfonyl, (C₁-C₄)alkylsulfonamido, amino, mono-N- o,r di-N,N-(C₁-C₄)alkylamino, carbamoyl, mono-N- or di-N,N-(C₁-C₄)alkylcarbamoyl, cyano, thiol, (C₁-C₆)alkylthio, (C₁-C₆)alkylsulfinyl, (C₁-C₄)alkylsulfonyl or mono-N- or di-N,N-(C₁-C₄)alkylaminosulfinyl;
R¹, R² and R³, when containing an alkyl, alkenyl, alkylene or alkenylene moiety, are optionally straight or branched and are optionally mono-, di- or tri-substituted on carbon independently with halo or hydroxy; and
V is a bond, -CO- or (C₁-C₃)alkylene optionally mono- or di-substituted independently with hydroxy or fluoro;
and said EP₄ receptor selective agonist is selected from compounds of Formula V: 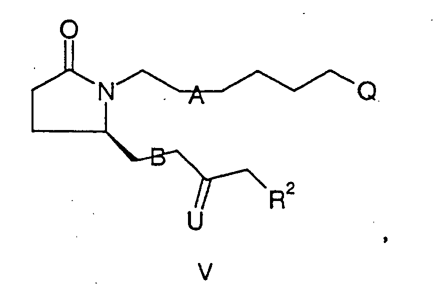 prodrugs thereof and pharmaceutically acceptable salts of said compounds or said prodrugs, wherein:
Q is COOR³, CONHR⁴ or tetrazol-5-yl;
A is a single or cis double bond;
B is a single or trans double bond;
U is 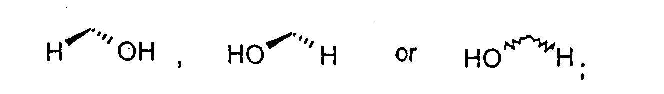
R² is α-thienyl, phenyl, phenoxy, monosubstituted phenyl and monosubstituted phenoxy, said substituents being chloro, fluoro, phenyl, methoxy, trifluoromethyl or (C₁-C₃)alkyl;
R³ is hydrogen, (C₁-C₅)alkyl, phenyl or p-biphenyl;
R⁴ is COR⁵ or SO₂R⁵; and
R⁵ is phenyl or C₁-C₅)alkyl.

3. The use as claimed in claims 1 to 2 wherein the condition is osteoporosis.

4. A pharmaceutical composition comprising an EP₂ receptor selective agonist as claimed in claims 1 and 2, an EP₄ receptor selective agonist as claimed in claims 1 and 2, and a pharmaceutically acceptable vehicle, carrier or diluent.

5. The use of an EP₂/EP₄ receptor selective agonist, prodrug or pharmaceutically acceptable salt thereof in the preparation of a medicament for the treatment of a condition which presents with low bone mass in a mammal.

6. The use as claimed in claim 5 where the EP₂/EP₄ receptor selective agonist is selected from compounds of the Formula VI: 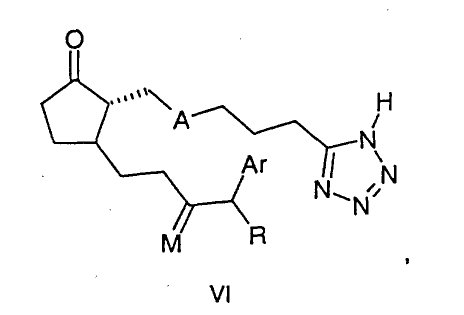 wherein A is ethylene or cis-vinylene; M is 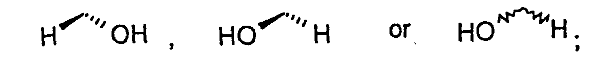 R is hydrogen or methyl; and Ar is phenyl, wherein said phenyl is optionally monosubstituted with fluoro, chloro, bromo, trifuoromethyl, methyl, methoxy or phenyl.

7. The use as claimed in claims 5 to 6 where the condition is osteoporosis.

8. The kit comprising
(a) a first dosage unit form comprising an EP₂ receptor selective agonist as claimed in claims 1 to 2 and a pharmaceutically acceptable vehicle, carrier or diluent.
(b) a second dosage unit form comprising an EP₄ receptor selective agonist as claimed in claims 1 to 2 and a pharmaceutically acceptable vehicle, carrier or diluent.
(c) a container where said dosage unit forms may be administered separately or together.
